(19) Europäisches Patentamt
European Patent Office
Office européen des brevets

(11) Publication number: **0 255 190**
**A2**

(12) **EUROPEAN PATENT APPLICATION**

(21) Application number: 87300397.4

(22) Date of filing: 19.01.87

(51) Int. Cl.³: C 12 N 15/00
C 12 N 1/20, C 12 P 21/02
C 07 K 13/00, G 01 N 33/569
G 01 N 33/543

(30) Priority: 01.08.86 US 892680

(43) Date of publication of application:
03.02.88 Bulletin 88/5

(84) Designated Contracting States:
BE CH DE ES FR GB GR IT LI NL SE

(71) Applicant: REPLIGEN CORPORATION
101 Binney Street
Cambridge Massachusetts 02142(US)

(72) Inventor: Putney, Scott D.
102 Gloucester Street
Arlington Massachusetts 02174(US)

(72) Inventor: Lynn, Debra
21 Allen Street, Apt. 11
Arlington Massachusetts 02174(US)

(72) Inventor: Javaherian, Kashayar
27 webster Road
Lexington Massachusetts 02173(US)

(72) Inventor: Mueller, william t.
26 Copeland Street
Watertown Massachusetts 02172(US)

(72) Inventor: farley, John
261 Culver Road No. 9
Rochester New York 14607(US)

(74) Representative: Perry, Robert Edward et al,
GILL JENNINGS & EVERY 53-64 Chancery Lane
London WC2A 1HN(GB)

(54) **Recombinant polypeptides and their uses, inclusing assay for aids virus.**

(57) Novel recombinant HTLV-III envelope proteins denoted R10, PB1, 590, and KH1, are useful in the diagnosis, prophylaxis or therapy of AIDS. Protein R10 is a 95 kD fusion protein; protein PB1 is a 26 kD fusion protein; protein 590 is an 86 kD fusion protein; and protein KH1 is a 70 kD fusion protein. These proteins are considered to be especially useful to prepare vaccines for the HTLV-III virus.

EP 0 255 190 A2

Croydon Printing Company Ltd

# RECOMBINANT POLYPEPTIDES AND THEIR USES, INCLUDING ASSAY FOR AIDS VIRUS

This invention relates to recombinant polypeptides and their uses, including assay for AIDS virus. The virus which causes AIDS (acquired immune deficiency syndrome) has been identified as human T-cell lymphotropic virus type III (HTLV-III), lymphadenopathy-associated virus (LAV) or AIDS-associated retrovirus (ARV); see Popovic et al, Science 224 (1984) 497-500. The virus has been designated HIV (human immunodeficiency virus) by the International Committee on Taxonomy of Virus.

The virus displays tropism for the $OKT4^+$ lymphocyte subset; see Klatzmann et al, Science 225 (1984) 59-63.

Antibodies against HTLV-III proteins in the sera of most AIDS and AIDS-related complex (ARC) patients, and in asymptomatic people infected with the virus (Sarngadharan et al, Science 224 (1984) 506-508) have made possible the development of immunologically based tests that detect antibodies to these antigens. These tests are used to limit the spread of HTLV-III through blood transfusion by identifying blood samples of people infected with the virus. Diagnostic tests currently available commercially use the proteins of inactivated virus as antigens.

In addition to allowing new approaches for diagnosis, recombinant DNA holds great promise for the development of vaccines against both bacteria and viruses; see Wilson, Bio/Technology 2 (1984) 29-39.

The most widely employed organisms to express recombinant vaccines have been E. coli, S. cerevisiae and cultured mammalian cells. For example, subunit vaccines against foot and mouth disease (Kleid, D.G., Yansura, D., Small, B., Dowbenko, D., Moore, D.M., Brubman, M.J., McKercher, P.D., Morgan, D.O., Robertson, B.H. and Bachrach, H.L. [1981] Science 214:1125-1129) and malaria (Young, J.F., Hockmeyer, W.T., Gross, M., Ripley Ballou, W., Wirtz, R.A., Trosper, J.H., Beaudoin, R.L., Hollingdale, M.R., Miller, L.M., Diggs, C.L. and Rosenberg, M. [1985] Science 228:958-962) have been synthesized in E. coli. Other examples are hepatitis B surface antigen produced in yeast (McAleer, W.J., Buynak, E.B., Maigetter, R.Z., Wampler, D.E., Miller, W.J. and Hilleman, M.R. [1984] Nature 307: 178-180) and a herpes vaccine produced in mammalian cells (Berman, P.W., Gregory, T., Chase, D. and Lasky, L.A. [1984] Science 227:1490-1492).

There is a real need at this time to develop a vaccine for AIDS. No such vaccine is known to exist.

## Brief Summary of the Invention

The subject invention concerns novel recombinant HTLV-III proteins and the uses thereof. More specifically, the subject invention concerns four novel recombinant HTLV-III envelope proteins which can be used in the diagnosis, prophylaxis or therapy of AIDS. Further, the recombinant HTLV-III envelope protein fragments of the invention can be used to stimulate a lymphocyte proliferative response in HTLV-III infected humans. This then would stimulate the immune system to respond to HTLV-III in such individuals and, therefore, the envelope protein fragments can provide protection and be of therapeutic value.

These novel proteins are encoded on bacterial plasmids which can be used to transform suitable hosts, for example, E. coli, using standard procedures.

In the accompanying drawings:

Figures 1A and 1B are sequential flow charts of the construction, from plasmid pBG1, of plasmid pREV2.2 which is used to construct vectors encoding novel proteins;

Figure 2 is a diagram of plasmid pREV2.2 and also of the multiple cloning site; and

Figure 3 is a schematic representation of the HTLV-III envelope gene and also of novel recombinant proteins obtained therefrom.

Expression vector plasmid pREV2.2 was constructed from plasmid pBG1 by the route shown in Figure 1 of the drawings. In the product, the hatched region represents TrpA trasc. terminator and the dotted region represents multiple cloning sites for enzymes NruI, MluI, EcoRV, ClaI, BamHI, SalI, HindIII, SmaI, StsI, EcoRI.

Plasmid pR10 contains approximately 1275 base pairs of DNA encoding the HTLV-III env gene from essentially the KpnI site to the BglII. This plasmid in a suitable bacterial host, e.g. E. coli, can be used to produce the novel recombinant HTLV-III 95 kD fusion protein denoted R10. The amino-acid sequence of fusion protein R10 is shown in Table 8; the DNA sequence encoding this protein is shown in Table 8A.

Plasmid pPB1 contains approximately 540 base pairs of DNA encoding essentially the HTLV-III env gene from the PvuII site to the BglII site. This plasmid in a suitable host, e.g. E. coli, can be used to produce the novel recombinant HTLV-III 26 kD fusion protein denoted PB1. The amino-acid sequence of fusion protein PB1 is shown in Table 9; the DNA sequence encoding this protein is shown in Table 9A.

Plasmid p590 contains approximately 1055 base pairs of DNA encoding essentially the HTLV-III env gene from the PvuII site to the HindIII site. This plasmid in a suitable host, e.g., E. coli, can be used to produce the novel recombinant HTLV-III 86 kD protein denoted 590. The amino acid sequence of fusion protein 590 is shown in Table 10; the DNA sequence encoding this protein is shown in Table 10A.

Plasmid pKH1 contains approximately 1830 base pairs of DNA encoding essentially the HTLV-III env gene from the KpnI site to the HindIII site. This plasmid in a suitable host, e.g., E. coli, can be used to produce the novel recombinant HTLV-III 70 kD protein denoted KH1. The amino acid sequence of fusion protein KH1 is shown in Table 11; the DNA sequence encoding this protein is shown in Table 11A.

Plasmid pBG1 is deposited in the E. coli host MS371 with the Northern Regional Research Laboratory (NRRL), U.S. Department of Agriculture, Peoria, Illinois, USA. It is in the permanent collection of this repository. E. coli MS371(pBG1), NRRL B-15904, was deposited on Nov. 1, 1984. E. coli MS371, NRRL B-15129, is now available to the public. E. coli SG20251, NRRL B-15918, was deposited on Dec. 12, 1984.

Other relevant NRRL deposits, their deposit dates and numbers, are as follows:

| Culture | Accession No. | Date of Deposit |
|---|---|---|
| E. coli JM103(pREV2.2) | NRRL B-18091 | July 30, 1986 |
| E. coli SG20251(pR10) | NRRL B-18093 | July 30, 1986 |
| E. coli SG20251(pPB1) | NRRL B-18092 | July 30, 1986 |
| E. coli SG20251(p590) | NRRL B-18094 | July 30, 1986 |
| E. coli CAG629(pKH1) | NRRL B-18095 | July 30, 1986 |

The novel HTLV-III proteins of the subject invention can be expressed in *Saccharomyces cerevisiae* using plasmids containing the inducible galactose promoter from this organism (Broach, J.R., Li, Y., Wu, L.C. and Jayaram, M. in Experimental Manipulation of Gene Expression [1983] p. 83, ed. M. Inouye, Academic Press). These plasmids are called YEp51 and YEp52 (Broach, J.R. et al. [1983]) and contain the *E. coli* origin of replication, the gene for β-lactamase, the yeast LEU2 gene, the 2 μm origin of replication and the 2 μm circle REP3 locus. Recombinant gene expression is driven by the yeast GAL10 gene promoter.

Yeast promoters such as galactose and alcohol dehydrogenase (Bennetzen, J.L. and Hall, B.D. [1982] J. Biol. Chem. 257:3018; Ammerer, G. in Methods in Enzymology [1983] Vol. 101, p. 192), phosphoglycerate kinase (Derynck, R., Hitzeman, R.A., Gray, P.W., Goeddel, D.V., in Experimental Manipulation of Gene Expression [1983] p. 247, ed. M. Inouye, Academic Press), triose phosphate isomerase (Alber, T. and Kawasaki, G. [1982] J. Molec. and Applied Genet. 1:419), or enolase (Innes, M.A. et al. [1985] Science 226:21) can be used.

The genes disclosed herein can be expressed in simian cells. When the genes encoding these proteins are cloned into one of the plasmids as described in Okayama and Berg (Okayama, H. and Berg, P. [1983] Molec. and Cell. Biol. 3:280) and references therein, or COS cells transformed with these plasmids, synthesis of HTLV-III proteins can be detected immunologically.

Other mammalian cell gene expression/protein production systems can be used. Examples of other such systems are the vaccinia virus expression system (Moss, B. [1985] Immunology Today 6:243; Chakrabarti, S., Brechling, K., Moss, B. [1985] Molec. and Cell. Biol. 5:3403) and the vectors derived from murine retroviruses (Mulligan, R.C. in Experimental Manipulation of Gene Expression [1983] p. 155, ed. M. Inouye, Academic Press).

The HTLV-III proteins of the subject invention can be chemically synthesized by solid phase peptide synthetic techinques such as BOC and FMOC (Merrifield, R.B. [1963] J. Amer. Chem. Soc. 85:2149; Chang, C. and Meienhofer, J. [1978] Int. J. Peptide Protein Res. 11:246).

As is well known in the art, the amino acid sequence of a protein is determined by the nucleotide sequence of the DNA. Because of the redundancy of the genetic code, i.e., more than one coding nucleotide triplet (codon) can be used for most of the amino acids used to make proteins, different nucleotide sequences can code for a particular amino acid. Thus, the genetic code can be depicted as follows:

| | | | |
|---|---|---|---|
| Phenylalanine (Phe) | TTK | Histidine (His) | CAK |
| Leucine (Leu) | XTY | Glutamine (Gln) | CAJ |
| Isoleucine (Ile) | ATM | Asparagine (Asn) | AAK |
| Methionine (Met) | ATG | Lysine (Lys) | AAJ |
| Valine (Val) | GTL | Aspartic acid (Asp) | GAK |
| Serine (Ser) | QRS | Glutamic acid (Glu) | CAJ |
| Proline (Pro) | CCL | Cysteine (Cys) | TGK |
| Threonine (Thr) | ACL | Tryptophan (Trp) | TGG |
| Alanine (Ala) | GCL | Arginine (Arg) | WGZ |
| Tyrosine (Tyr) | TAK | Glycine (Gly) | GGL |
| Termination Signal | TAJ | | |
| Termination Signal | TGA | | |

Key: Each 3-letter deoxynucleotide triplet corresponds to a trinucleotide of mRNA, having a 5'-end on the left and a 3'-end on the right. All DNA sequences given herein are those of the strand whose sequence corresponds to the mRNA sequence, with thymine substituted for uracil. The letters stand for the purine or pyrimidine bases forming the deoxynucleotide sequence.

A = adenine
G = guanine
C = cytosine
T - thymine
X = T or C if Y is A or G
X = C if Y is C or T
Y = A, G, C or T if X is C
Y = A or G if X is T
W = C or A if Z is A or G
W = C if Z is C or T
Z = A, G, C or T if W is C
Z = A or G if W is A
QR = TC if S is A, G, C or T; alternatively QR = AG if S is T or C

J = A or G
K = T or C
L = A, T, C or G
M = A, C or T

The above shows that the novel amino acid sequences of the HTLV-III proteins of the subject invention can be prepared by nucleotide sequences other than those disclosed herein. Functionally equivalent nucleotide sequences encoding the novel amino acid sequences of these HTLV-III proteins, or fragments thereof having HTLV-III antigenic or immunogenic or therapeutic activity, can be prepared by known synthetic procedures. Accordingly, the subject invention includes such functionally equivalent nucleotide sequences.

Thus the scope of the subject invention includes not only the specific nucleotide sequences depicted herein, but also all equivalent nucleotide sequences coding for molecules with substantially the same HTLV-III antigenic or immunogenic or therapeutic activity.

Further, the scope of the subject invention is intended to cover not only the specific amino acid sequences disclosed, but also similar sequences coding for proteins or protein fragments having comparable ability to induce the formation of and/or bind to specific HTLV-III antibodies.

The term "equivalent" is being used in its ordinary patent usage here as denoting a nucleotide sequence which performs substantially as the nucleotide sequence identified herein to produce molecules with substantially the same HTLV-III antigenic or immunogenic or therapeutic activity in essentially the same kind of hosts. Within this definition are subfragments which have HTLV-III antigenic or immunogenic or therapeutic activity.

As disclosed above, it is well within the skill of those in the genetic engineering art to use the nucleotide sequences encoding HTLV-III antigenic or immunogenic or therapeutic activity of the subject invention to produce HTLV-III proteins via microbial processes. Fusing the sequences into an expression vector and transforming or transfecting into hosts, either eukaryotic (yeast or mammalian cells) or prokaryotic (bacterial cells), are standard procedures used in producing other well-known proteins, e.g., insulin, interferons, human growth hormone, IL-1, IL-2, and the like. Similar procedures, or obvious modifications thereof, can be employed to prepare HTLV-III proteins by microbial means or tissue-culture technology in accord with the subject invention.

The nucleotide sequences disclosed herein can be prepared by a "gene machine" by procedures well known in the art. This is possible because of the disclosure of the nucleotide sequence.

The restriction enzymes disclosed can be purchased from Bethesda Research Laboratories, Gaithersburg, MD, or New England Biolabs, Beverly, MA. The enzymes are used according to the instructions provided by the supplier.

The various methods employed in the preparation of the plasmids and transformation of host organisms are well known in the art. These procedures are all described in Maniatis, T., Fritsch, E.F., and Sambrook, J. (1982) Molecular Cloning: A Laboratory Manual, Cold Spring Harbor Laboratory, New York. Thus, it is within the skill of those in the genetic engineering art to extract DNA from microbial cells, perform restriction enzyme digestions, electrophorese DNA fragments, tail and anneal plasmid and insert DNA, ligate DNA, transform cells, e.g., *E. coli* cells, prepare plasmid DNA, electrophorese proteins, and sequence DNA.

Immunochemical assays employing the HTLV-III proteins of the invention can take a variety of forms. The preferred type is a solid phase immunometric assay. In assays of this type, an HTLV-III protein is immobilized on a solid phase to form an antigen-immunoadsorbent. The immunoadsorbent is incubated with the sample to be tested. After an appropriate incubation period, the immunoadsorbent is separated from the sample and labeled anti-(human IgG) antibody is used to detect human anti-HTLV-III antibody bound to the immunoadsorbent. The amount of label associated with the immunoadsorbent can be compared to positive and negative controls to assess the presence or absence of anti-HTLV-III antibody.

The immunoadsorbent can be prepared by adsorbing or coupling a purified HTLV-III protein to a solid phase. Various solid phases can be used, such as beads formed of glass, polystyrene, polypropylene, dextran or other material. Other suitable solid phases include tubes or plates formed from or coated with these materials.

The HTLV-III proteins can be either covalently or non-covalently bound to the solid phase by techniques such as covalent bonding via an amide or ester linkage or adsorption. After the HTLV-III protein is affixed to the solid phase, the solid phase can be post-coated with an animal protein, e.g., 3% fish gelatin. This provides a blocking protein which reduces nonspecific adsorption of protein to the immunoadsorbent surface.

The immunoadsorbent is then incubated with the sample to be tested for anti-HTLV-III antibody. In blood screening, blood plasma or serum is used. The plasma or serum is diluted with normal animal plasma or serum. The diluent plasma or serum is derived from the same animal species that is the source of the anti-(human IgG) antibody. The preferred anti-(human IgG) antibody is goat anti-(human IgG) antibody. Thus, in the preferred format, the diluent would be goat serum or plasma.

The conditions of incubation, e.g., pH and temperature, and the duration of incubation are not crucial. These parameters can be optimized by routine experimentation. Generally, the incubation will be run for 1-2 hr at about 45°C in a buffer of pH 7-8.

After incubation, the immunoadsorbent and the sample are separated. Separation can be accomplished by any conventional separation technique such as sedimentation or centrifugation. The immunoadsorbent then may be washed free of sample to eliminate any interfering substances.

The immunoadsorbent is incubated with the labeled anti-(human IgG) antibody (tracer) to detect human antibody bound thereto. Generally the immunoadsorbent is incubated with a solution of the labeled anti-(human IgG) antibody which contains a small amount (about 1%) of the serum or plasma of the animal species which serves as the source of the anti-(human IgG) antibody. Anti-(human IgG) antibody can be obtained from any animal source. However, goat anti-(human IgG) antibody is preferred. The anti-(human IgG) antibody can be an antibody against the Fc fragment of human IgG, for example, goat anti-(human IgG) Fc antibody.

The anti-(human IgG) antibody or anti-(human IgG)Fc can be labeled with a radioactive material such as $^{125}$iodine; labeled with an optical label, such as a fluorescent material; or labeled with an enzyme such as horseradish peroxidase. The anti-human antibody can also be biotinylated and labeled avidin used to detect its binding to the immunoadsorbent.

After incubation with the labeled antibody, the immunoadsorbent is separated from the solution and the label associated with the immunoadsorbent is evaluated. Depending upon the choice of label, the evaluation can be done in a variety of ways. The label may be detected by a gamma counter if the label is a radioactive gamma emitter, or by a fluorimeter, if the label is a fluorescent material. In the case of an enzyme, label detection may be done colorimetrically employing a substrate for the enzyme.

The amount of label associated with the immunoadsorbent is compared with positive and negative controls in order to determine the presence of anti-HTLV-III antibody. The controls are generally run concomitantly with the sample to be tested. A positive control is a serum containing antibody against HTLV-III; a negative control is a serum from healthy individuals which does not contain antibody against HTLV-III.

For convenience and standardization, reagents for the performance of the immunometric assay can be assembled in assay kits. A kit for screening blood, for example, can include:

(a) an immunoadsorbent, e.g., a polystyrene bead coated with an HTLV-III protein;

(b) a diluent for the serum or plasma sample, e.g., normal goat serum or plasma;

(c) an anti-(human IgG) antibody, e.g., goat anti-(human IgG) antibody in buffered, aqueous solution containing about 1% goat serum or plasma;

(d) a positive control, e.g., serum containing antibody against at least one of the novel HTLV-III proteins; and

(e) a negative control, e.g., pooled sera from healthy individuals which does not contain antibody against at least one of the novel HTLV-III proteins.

If the label is an enzyme, an additional element of the kit can be the substrate for the enzyme.

Another type of assay for anti-HTLV-III antibody is an antigen sandwich assay. In this assay, a labeled HTLV-III protein is used in place of anti-(human IgG) antibody to detect anti-HTLV-III antibody bound to the immunoadsorbent. The assay is based in principle on the bivalency of antibody molecules. One binding site of the antibody binds the antigen affixed to the solid phase; the second is available for binding the labeled antigen. The assay procedure is essentially the same as described for the immunometric assay except that after incubation with the sample, the immunoadsorbent is incubated with a solution of labeled HTLV-III protein. HTLV-III proteins can be labeled with radioisotope, an enzyme, etc. for this type of assay.

In a third format, the bacterial protein, protein A, which binds the Fc segment of an IgG molecule without interfering with the antigen-antibody interaction can be used as the labeled tracer to detect anti-HTLV-antibody adsorbed to the immunoadsorbent. Protein A can be readily labeled with a radioisotope, enzyme or other detectable species.

Immunochemical assays employing an HTLV-III protein have several advantages over those employing a whole (or disrupted) virus. Assays based upon an HTLV-III protein will alleviate the concern over growing large quantities of infectious virus and the inherent variability associated with cell culturing and virus production. Further, the assay will help mitigate the real or perceived fear of contracting AIDS by technicians in hospitals, clinics and blood banks who perform the test.

Vaccines of the HTLV-III proteins, disclosed herein, and variants thereof having antigenic properties, can be prepared by procedures well known in the art. For example, such vaccines can be prepared as injectables, e.g., liquid solutions or suspensions. Solid forms for solution in, or suspension in, a liquid prior to injection also can be prepared. Optionally, the preparation also can be emulsified. The active antigenic ingredient or ingredients can be mixed with excipients which are pharmaceutically acceptable and compatible with the active ingredient. Examples of suitable excipients are water, saline, dextrose, glycerol, ethanol, or the like, and combinations thereof. In addition, if desired, the vaccine can contain minor amounts of auxiliary substances such as wetting or emulsifying agents, pH buffering agents, or adjuvants which enhance the effectiveness of the vaccine. The vaccines are conventionally administered parenterally, by injection, for example, either subcutaneously or intramuscularly. Additional formulations which are suitable for other modes of administration include suppositories and, in some cases, oral formulations.

For suppositories, traditional binders and carriers include, for example, polyalkalene glycols or triglycerides. Suppositories can be formed from mixtures containing the active ingredient in the range of about 0.5% to about 10%, preferably about 1 to about 2%. Oral formulations can include such normally employed excipients as, for example, pharmaceutical grades of manitol, lactose, starch, magnesium stearate, sodium saccharine, cellulose, magnesium carbonate and the like. These compositions can take the form of solutions, suspensions, tablets, pills, capsules, sustained release formulations or powders and contain from about 10% to about 95% of active ingredient, preferably from about 25% to about 70%.

The proteins can be formulated into the vaccine as neutral or salt forms. Pharmaceutically acceptable salts include the acid addition salts (formed with the free amino groups of the peptide) and which are formed with inorganic acids such as, for example, hydrochloric or phosphoric acids, or such organic acids as acetic, oxalic, tartaric, mandelic, and the like. Salts formed with the free carboxyl groups also can be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be therapeutically effective and immunogenic. The quantity to be administered depends on the subject to be treated, capacity of the subject's immune system to synthesize antibodies, and the degree of protection desired. Precise amounts of active ingredient required to be administered depend on the judgment of the practitioner and are peculiar to each individual. However, suitable dosage ranges are of the order of about several hundred micrograms active ingredient per individual. Suitable regimes for initial administration and booster shots are also variable, but are typified by an initial administration followed in one or two week intervals by a subsequent injection or other administration.

HTLV-III is known to undergo amino acid sequence variation, particularly in the envelope gene (Starcich, B.R. [1986] Cell 45:637-648; Hahn, B.H. et al. [1986] Science 232:1548-1553). Over 100 variants have been analyzed by molecular cloning and restriction enzyme recognition analysis, and several of these have been analyzed by nucleotide sequencing. Some of these are the HTLV-III isolates known as RF (Popovic, M. et al. [1984] Science 224:497-500), WMJ-1 (Hahn, B.H. et al. [1986] Science 232:1548-1553), LAV (Wain-Hobson, S. et al. [1985] Cell 40:9-17), and ARV-2 (Sanchez-Pescador, R. et al. [1985] Science 227:484-492). Although the subject invention describes the sequence from one HTLV-III isolate, the appropriate envelope regions of any HTLV-III isolate can be produced using the procedures described herein for preparing R10, PB1, 590, and KH1. The HTLV-III proteins from different viral isolates can be used in vaccine preparations, as disclosed above, to protect against infections by different HTLV-III isolates. Further, a vaccine preparation can be made using more than one recombinant antigenic protein from more than one HTLV-III isolate to provide immunity and thus give better protection against AIDS.

Following are examples which illustrate the process of the invention, including the best mode. These examples should not be construed as limiting. All solvent mixture proportions are by volume unless otherwise noted.

Example 1--Construction of plasmid pREV2.2

The pREV2.2 plasmid expression vector was constructed from plasmid pBG1. Plasmid pBG1 can be isolated from its E. coli host by well known procedures, e.g., using cleared lysate-isopycnic density gradient procedures, and the like. Like pBG1, pREV2.2 expresses inserted genes behind the E. coli promoter. The differences between pBG1 and pREV2.2 are the following:

1. pREV2.2 lacks a functional replication of plasmid (rop) protein.
2. pREV2.2 has the trpA transcription terminator inserted into the AatII site. This sequence insures transcription termination of over-expressed genes.

3. pREV2.2 has genes to provide resistance to ampicillin and chloramphenicol, whereas pBG1 provides resistance only to ampicillin.
4. pREV2.2 contains a sequence encoding sites for several restriction endonucleases.

The following procedures, shown in Figure 1 of the drawings, were used to make each of the four changes listed above:

1a. 5 μg of plasmid pBG1 was restricted with NdeI which gives two fragments of approximately 2160 and 3440 base pairs.

1b. 0.1 μg of DNA from the digestion mixture, after inactivation of the NdeI, was treated with T4 DNA ligase under conditions that favor intramolecular ligation (200 μl reaction volume using standard T4 ligase reaction conditions [New England Biolabs, Beverly, MA]). Intramolecular ligation of the 3440 base pair fragment gave an ampicillin resistant plasmid. The ligation mixture was transformed into the recipient strain E. coli JM103 (available from New England Biolabs) and ampicillin resistant clones were selected by standard procedures.

1c. The product plasmid, pBG1ΔN, where the 2160 base pair NdeI fragment is deleted from pBG1, was selected by preparing plasmid from ampicillin resistant clones and determining the restriction digestion patterns with NdeI and SalI (product fragments approximately 1790 and 1650). This deletion inactivates the rop gene that controls plasmid replication.

2a. 5 µg of pBG1ΔN was then digested with EcoRI and BclI and the larger fragment, approximately 2455 base pairs, was isolated.

2b. A synthetic double stranded fragment was prepared by the procedure of Itakura et al. (Itakura, K., Rossi, J.J. and Wallace, R.B. [1984] Ann. Rev. Biochem. 53:323-356, and references therein) with the structure shown in Table 1. This fragment has BclI and EcoRI sticky ends and contains recognition sequences for several restriction endonucleases.

2c. 0.1 µg of the 2455 base pair EcoRI-BclI fragment and 0.01 µg of the synthetic fragment were joined with T4 DNA ligase and competent cells of strain JM103 were transformed. Cells harboring the recombinant plasmid, where the synthetic fragment was inserted into pBG1ΔN between the BclI and EcoRI sites, were selected by digestion of the plasmid with HpaI and EcoRI. The diagnostic fragment sizes are approximately 2355 and 200 base pairs. This plasmid is called pREV1.

2d. 5 µg of pREV1 were digested with AatII, which cleaves uniquely.

2e. The double stranded fragment shown in Table 2 was synthesized. This fragment has AatII sticky ends and contains the trpA transcription termination sequence.

2f. 0.1 µg of AatII digested pREV1 was ligated with 0.01 µg of the synthetic fragment in a volume of 20 µl using T4 DNA ligase.

2g. Cells of strain JM103, made competent, were transformed and ampicillin resistant clones selected.

2h. Using a KpnI, EcoRI double restriction digest of plasmid isolated from selected colonies, a cell containing the correct construction was isolated. The sizes of the KpnI, EcoRI generated fragments are approximately 2475 and 80 base pairs. This plasmid is called pREV1TT and contains the trpA transcription terminator.

3a. 5 µg of pREV1TT, prepared as disclosed above (by standard methods) was cleaved with NdeI and XmnI and the approximately 850 base pair fragment was isolated.

3b. 5 µg of plasmid pBR325 (BRL, Gaithersburg, MD), which contains the genes conferring resistance to chloramphenicol as well as to ampicillin and tetracycline, was cleaved with BclI and the ends blunted with Klenow polymerase and deoxynucleotides. After inactivating the enzyme, the mixture was treated with NdeI and the approximately 3185 base pair fragment was isolated. This fragment contains the genes for chloramphenicol and ampicillin resistance and the origin of replication.

3c. 0.1 µg of the NdeI-XmnI fragment from pREV1TT and the NdeI-BclI fragment from pBR325 were ligated in 20 µl with T4 DNA ligase and the mixture used to transform competent cells of strain JM103. Cells resistant to both ampicillin and chloramphenicol were selected.

3d. Using an EcoRI and NdeI double digest of plasmid from selected clones, a plasmid was selected giving fragment sizes of approximately 2480, 1145, and 410 base pairs. This is called plasmid pREV1TT/chl and has genes for resistance to both ampicillin and chloramphenicol.

4a. A double stranded fragment shown in Table 3 was synthesized. This fragment, with a blunt end and an SstI sticky end, contains recognition sequences for several restriction enzyme sites.

4b. 5 µg of pREV1TT/chl was cleaved with NruI (which cleaves about 20 nucleotides from the BclI site) and SstI (which cleaves within the multiple cloning site). The larger fragment, approximately 3990 base pairs, was isolated from an agarose gel.

4c. 0.1 µg of the NruI-SstI fragment from pREV1TT/chl and 0.01 µg of the synthetic fragment were treated with T4 DNA ligase in a volume of 20 µl.

4d. This mixture was transformed into strain JM103 and ampicillin resistant clones were selected.

4e. Plasmid was purified from several clones and screened by digestion with MluI or ClaI. Recombinant clones with the new multiple cloning site will give one fragment when digested with either of these enzymes, because each cleaves the plasmid once.

4f. The sequence of the multiple cloning site was verified. This was done by restricting the plasmid with HpaI and PvuII and isolating the 1395 base pair fragment, cloning it into the SmaI site of mp18 and sequencing it by dideoxynucleotide sequencing using standard methods.

4g. This plasmid, called pREV2.2 is diagrammed in Figure 2 of the drawings.

Example 2--Construction of and expression from pR10

Plasmid pR10, which contains approximately 1275 base pairs of DNA encoding the HTLV-III env gene from essentially the KpnI site to the BglII site, and from which is synthesized an approximately 95 kD fusion protein containing this portion of the gp120 envelope protein, can be constructed as follows:

1. Synthesizing the DNA with the sequence shown in Table 4. This DNA fragment can be synthesized by standard methods (Itakura, et al., supra, and references therein) and encodes a portion of gp120. It has a blunt end on the 5' end and an end which will ligate with a BamHI overhang on the 3' end.
2. Restricting 5 μg of plasmid pBG1 with BclI, filling in the overhanging ends with Klenow polymerase and deoxyribonucleotide triphosphates (dNTPs), restricting this fragment with BamHI and isolating the large fragment, approximately 8.9 kb, from an agarose gel.
3. Ligating 0.1 μg of the fragment in Table 4 with 0.1 μg of the pBG1 fragment in a volume of 20 μl using T4 DNA ligase, transforming the ligation mixture into competent cell strain SG20251 (Gottesman, S., Halpern, E. and Trisler, P. [1981] Journal of Bacteriology 148:265-273), and selecting ampicillin resistant transformants.

4. Selecting, using the AhaIII restriction pattern of purified plasmid, cells harboring the recombinant plasmid with the synthesized fragment in the orientation whereby the fragment blunt end ligated to the pBG1 fragment filled-in BclI end and the BamHI overhanging ends ligated together. AhaIII digestion of the proper plasmid gives fragment lengths of approximately 5300, 3170, 690, 640, 330, and 20 base pairs.
5. When the strain harboring this recombinant plasmid is grown in 2% medium (2% yeast extract, bactotryptone, casamino acids (Difco, Detroit, MI), 0.2% potassium monobasic, 0.2% potassium dibasic, and 0.2% sodium dibasic) containing 50 μg/ml ampicillin and the total complement of cellular proteins electrophoresed on an SDS-polyacrylamide gel, a prominent protein of approximately 95 kD can be visualized by either coomassie blue staining or by western blot analysis using as probe selected sera from AIDS, ARC, or HTLV-III infected individuals.

Example 3--Purification of recombinant protein containing HTLV-III envelope sequences from plasmid pR10

1. Growth of cells:

Cells were grown in a 10 liter volume in a Chemap fermentor (Chemapec, Woodbury, NY) in 2% medium. Fermentation temperature was 37°C, the pH was 6.8, and air was provided at 1 vvm. Plasmid selection was provided by 50 μg/ml ampicillin. Typical cell yield (wet weight) is 30 g/l.

2. Cell lysis:

50 g, wet cell weight, of *E. coli* containing the recombinant HTLV-III envelope fusion protein were resuspended in a final volume of 100 ml in 50 mM Tris-Cl pH 8.0, 5 mM potassium ethylenediaminetetraacetic acid (KEDTA), 5 mM dithiothreitol (DTT), 15 mM β-mercaptoethanol, 0.5% Triton X-100, and 5 mM phenylmethylsulfonyl fluoride (PMSF). 300 mg lysozyme was added and the suspension incubated for 30 min at room temperature.

This material was lysed using a BEAD-BEATER™ (Biospec Products, Bartlesville, OK) containing an equal volumn of 0.1-0.15 μm glass beads. The lysis was done for 6 min at room temperature in 1 min intervals. The liquid was separated from the beads and centrifuged for 2.5 hr at 20,000 xg. The supernatant was removed and the pellet dissolved in 100 ml 8 M urea, 20 mM Tris-Cl pH 8.0, 5 mM DTT, 15 mM β-mercaptoethanol, 5 mM PMSF, and 1 mM KEDTA. The pellet was solubilized using a polytron homogenizer (Beckman, Berkeley, CA) and centrifuged at 20,000 xg for 2 hr.

3. Diethylaminoethyl (DEAE) chromatography:

Supernatant was loaded onto a 550 ml column (5 cm x 28 cm) packed with DEAE Fast Flow Sepharose (Pharmacia, Piscataway, NJ) equilibrated in 8 M urea, 20 mM Tris-Cl pH 8.0, 15 mM β-mercaptoethanol, and 1 mM KEDTA at room temperature. The column was washed with 1.5 liters equilibration buffer, and the protein eluted with a 5.0 liter linear gradient from 0-0.8 M NaCl in equilibration buffer. The HTLV-III protein eluted at 0.2 M NaCl and was assayed using SDS-polyacrylamide electrophoresis and following the prominent protein at approximately 95 kD.

The fractions containing the HTLV-III protein were pooled and the protein concentrated to 10 ml using a stressed cell positive pressure concentrator (Amicon, Danvers, MA) fitted with a 10,000 MW cut-off membrane (YM-10, Amicon). The concentrate was loaded onto a 500 ml column (2.5 cm x 100 cm) packed with superfine sephacryl S-300 (Pharmacia) equilibrated in 8 M urea, 20 mM Tris-Cl, pH 8.0, 15 mM β-mercaptoethanol, and 1 mM KEDTA. The column was eluted with equilibration buffer at room temperature. A flow rate of 0.5 ml/min was maintained. The HTLV-III protein eluted at approximately 40% of the column volume.

Example 4--Construction of and expression from plasmid pPB1

Plasmid pPB1, which contains approximately 540 base pairs of DNA encoding essentially the HTLV-III env gene from the PvuII site to the BglII site, and from which is synthesized an approximately 26 kD fusion protein containing this portion of the gp120 envelope protein can be constructed as follows:

1. Synthesizing the DNA with the sequence shown in Table 5: This DNA fragment can be synthesized by standard methods and encodes a portion of gp120. It has a blunt end on the 5' end and an end which will ligate with a BamHI overhang on the 3' end.
2. Restricting 5 μg plasmid pREV2.2 with EcoRV and BamHI and isolating the large fragment, approximately 4 kD, from an agarose gel.
3. Ligating 0.1 μg of the fragment in Table 5 with 0.1 μg of the pREV2.2 fragment in a volume of 20 μl using T4 DNA ligase, transforming the ligation mixture into competent cell strain SG20251, and selecting ampicillin resistant transformants.
4. Using the AhaIII restriction pattern of purified plasmid, selecting cells harboring the recombinant plasmid with the synthesized fragment in the orientation whereby the fragment blunt end ligated to the REV2.2 EcoRV end and the BamHI overhanging ends ligated together. AhaIII digestion of the proper plasmid gives fragment lengths of approximately 1210, 1020, 750, 690, 500, 340, and 20 base pairs. When the strain harboring this recombinant plasmid is grown in 2% medium containing 50 μg/ml ampicillin and the total complement of cellular proteins electrophoresed on an SDS-polyacrylamide gel, a protein of approximately 26 kD can be visualized by either coomassie blue staining or by western blot analysis using as probe selected sera from AIDS, ARC, or HTLV-III infected individuals.

Example 5--Purification of recombinant protein containing HTLV-III envelope sequences from plasmid pPB1

1. Growth of cells:

Cells were grown in a 10 liter volume in a Chemap fermentor in 2% medium. Fermentation temperature was 37°C, the pH was 6.8, and air was provided at 1 vvm. Plasmid selection was provided by 50 μg/ml ampicillin and 20 μg/ml chloramphenicol. Typical cell yield (wet weight) was 30 g/l.

2. Cell Lysis:

50 g, wet cell weight, of E. coli containing the recombinant HTLV-III envelope fusion protein were resuspended in a final volume of 100 ml in 50 mM Tris-Cl pH 8.0, 5 mM KEDTA, 5 mM DTT, 15 mM β-mercaptoethanol, 0.5% Triton X-100, and 5 mM PMSF. 300 mg lysozyme was added and the suspension incubated for 30 min at room temperature.

This material was lysed using a BEAD-BEATER™ (Biospec Products, Bartlesville, OK) containing an equal volume of 0.1-0.15 µm glass beads. The lysis was done for 6 min at room temperature in 1 min intervals. The liquid was separated from the beads and centrifuged for 2.5 hr at 20,000 xg. The supernatant was removed and the pellet was resuspended in 100 ml 6 M guanidine-hydrochloride, 20 mM Tris-Cl pH 8.0, 5 mM DTT, 15 mM β-mercaptoethanol, 5 mM PMSF, and 1 mM KEDTA. The pellet was solubilized using a polytron homogenizer and centrifuged at 20,000 xg for 2 hr.

The supernate (90 ml) was dialysed against 4 liters of 8 M urea, 20 mM Tris-Cl, pH 8.0, 1 mM EDTA, and 15 mM β-mercaptoethanol. Dialysis was done each time for 8 hr or longer with three changes of buffer. Spectraphor dialysis tubing (S/P, McGraw Park, IL) with a 3.5 kD MW cut-off was used.

3. CM chromatography

The dialysate was loaded onto a 550 ml column (5 cm x 28 cm) packed with CM Fast Flow Sepharose (Pharmacia) equilibrated in 8 M urea, 10 mM potassium phosphate pH 7.0, 15 mM β-mercaptoethanol, and 1 mM KEDTA at room temperature. The column was washed with 2 liters equilibration buffer, and the protein eluted with a 5.0 liter linear gradient from 0-0.4 M NaCl. The HTLV-III protein (26 kD) eluted at approximately 0.2 M NaCl as assayed by SDS-polyacrylamide gel electrophoresis.

Example 6--Construction of and expression from plasmid p590

Plasmid p590, which contains approximately 1055 base pairs of DNA encoding essentially the HTLV-III env gene from the PvuII site to the HindIII site, and from which is synthesized an approximately 86 kD fusion protein containing this portion of the gp160 envelope protein can be constructed as follows:

1. Synthesizing the DNA with the sequence shown in Table 6: This DNA fragment can be synthesized by standard methods and encodes a portion of gp160. It has a blunt end on the 5' end and an end which will ligate with a HindIII overhang on the 3' end.
2. Restricting 5 μg plasmid pREV2.2 with EcoRV and HindIII and isolating the large fragment, approximately 4 kD, from an agarose gel.
3. Ligating 0.1 μg of the fragment in Table 6 with 0.1 μg of the pREV2.2 fragment in a volume of 20 ml using T4 DNA ligase, transforming the ligation mixture into competent cell strain SG20251, and selecting ampicillin resistant transformants.
4. Using the AhaIII restriction pattern of purified plasmid, selectiong cells harboring the recombinant plasmid with the synthesized fragment in the orientation whereby the fragment blunt end ligated to the pREV2.2 EcoRV end and the HindIII overhanging ends ligated together. AhaIII digestion of the proper plasmid gives fragment lengths of approximately 1740, 1020, 750, 690, 500, 340, and 20.
5. 5 μg of plasmid, purified from this strain, is restricted with NdeI and SmaI. The approximately 1425 base pair fragment is isolated from an agarose gel. The 1505 base pair fragment is fused to the DNA encoding the segment of gp160.
6. 5 μg of pBG101 is restricted with BamHI, the overhanging ends filled in with Klenow polymerase and dNTPs, and then restricted with NdeI. The approximately 6.5 kD fragment is isolated from an agarose gel.

7. Ligating 0.1 μg of the NdeI-SmaI fragment with 0.1 μg of the pBG1 fragment using T4 DNA ligase, transforming the ligation mixture into competent cell strain SG20251, and selecting ampicillin resistant transformants.
8. Using the AhaIII restriction pattern of purified plasmid, selecting cells harboring the recombinant plasmid with the synthesized fragment in the orientation whereby the fragment blunt SmaI end ligated to the BamHi/filled-in end and the NdeI overhanging ends ligated together. AhaIII digestion of the proper plasmid gives fragment lengths of approximately 5900, 1020, 690, 430, and 20 base pairs.
9. When the strain harboring this recombinant plasmid is grown in 2% medium containing 50 μg/ml ampicillin and the total complement of cellular proteins electrophoresed on an SDS-polyacrylamide gel, a protein of approximately 86 kD can be visualized by either coomassie blue staining or by western blot analysis using as probe selected sera from AIDS, ARC, or HTLV-III infected individuals.

Example 7--Purification of recombinant protein containing HTLV-III envelope sequences from plasmid p590

1. Growth of cells:

Cells were grown in a 10 liter volume in a Chemap fermentor in 2% medium. Fermentation temperature was 37°C, the pH was 6.8, and air was provided at 1 vvm. Plasmid selection was provided by 50 μg/ml ampicillin. Typical cell yield (wet weight) is 30 g/l.

2. Cell Lysis:

50 g, wet cell weight, of *E. coli* containing the recombinant HTLV-III envelope fusion protein were resuspended in a final volume of 100 ml in 50 mM Tris-Cl pH 8.0, 5 mM KEDTA, 5 mM DTT, 15 mM β-mercaptoethanol. 0.5% Triton X-100, and 5 mM PMSF. 300 mg lysozyme was added and the suspension incubated for 30 min at room temperature.

This material was lysed using a Bead-Beater™ containing 0.1-0.15 mm glass beads. The lysis was done for 6 min at room temperature in 1 min intervals. The liquid was separated from the beads and centrifuged for 2.5 hr at 20,000 xg. The supernatant was removed and the pellet was resuspended in 100 ml 6 M guanidine-hydrochloride, 20 mM Tris-Cl pH 8.0, 5 mM DTT, 15 mM β-mercaptoethanol, 5 mM PMSF, and 1 mM KEDTA. The pellet was solubilized using a polytron homogenizer and centrifuged at 20,000 xg for 2 hr.

The supernate (90 ml) was dialysed against 4 liters of 8 M urea, 20 mM Tris-Cl. pH 8.0, 1 mM EDTA, and 15 mM β-mercaptoethanol. Dialysis was done each time for 8 hr or longer with three changes of buffer. Spectraphor dialysis tubing with a 3.5 kD MW cut-off was used.

3. Diethylaminoethyl (DEAE) chromatography

Dialysate was loaded onto a 550 ml column (5 cm x 28 cm) packed with DEAE Fast Flow Sepharose (Pharmacia) equilibrated in 8 M urea, 20 mM Tris-Cl pH 8.0, 15 mM β-mercaptoethanol, and 1 mM KEDTA at room temperature. The column was washed with 1.5 liters equilibration buffer, and the protein eluted with a 5.0 liter linear gradient from 0-0.8 M NaCl in equilibration buffer. The HTLV-III protein eluted at 0.4 M NaCl and was assayed using SDS-polyacrylamide electrophoresis and following the prominent protein at approximately 86 kD.

The fractions containing the HTLV-III protein were pooled and the protein concentrated to 10 ml using a stressed cell positive pressure concentrator (Amicon) fitted with a 10,000 MW cut-off membrane (YM-10, Amicon). The concentrate was loaded onto a 500 ml column (2.5 cm x 100 cm) packed with superfine sephacryl S-300 (Pharmacia) equilibrated in 8 M urea, 20 mM Tris-Cl, pH 8.0, 15 mM β-mercaptoethanol, and 1 mM KEDTA. The column was eluted with equilibration buffer at room temperature. A flow rate of 0.5 ml/min was maintained. The HTLV-III protein eluted at approximately 40% of the column volume.

Example 8--Construction of and expression from plasmid pKH1

Plasmid pKH1, which contains approximately 1830 base pairs of DNA encoding essentially the HTLV-III env gene from the KpnI site to the HindIII site, and from which is synthesized an approximately 70 kD fusion protein containing this portion of the gp160 envelope protein, can be constructed as follows:

1. Synthesizing the DNA with the sequence shown in Table 7: This DNA fragment can be synthesized by standard methods and encodes a portion of gp160. It has a blunt end on the 5' end and an end which will ligate with a HindIII overhang on the 3' end.
2. Restricting 5 μg plasmid pREV2.2 with MluI, treating the DNA with Klenow polymerase to blunt the ends, treating with HindIII and isolating the large fragment, approximately 5 kD, from an agarose gel.
3. Ligating 0.1 μg of the fragment in Table 7 with 0.1 μg of the pREV 2.2 fragment in a volume of 20 µl using T4 DNA ligase, transforming the ligation mixture into competent cell strain CAG629, and selecting ampicillin resistant transformants.

4. Using the AhaIII restriction pattern of purified plasmid, selecting cells harboring the recombinant plasmid with the synthesized fragment in the orientation whereby the fragment blunt end ligated to the REV2.2 MluI end and the HindIII overhanging ends ligated together. AhaIII digestion of the proper plasmid gives fragment lengths of approximately 1730, 1020, 750, 690, 640, 600, 340, and 20 base pairs. When the strain harboring this recombinant plasmid is grown in 2% medium containing 50 µg/ml ampicillin and the total complement of cellular proteins electrophoresed on an SDS-polyacrylamide gel, a protein of approximately 70 kD can be visualized by either Coomassie blue staining or by Western Blot analysis using as probe selected sera from AIDS, ARC, or HTLV-III infected individuals.

Example 9--Purification of recombinant protein containing HTLV-III envelope sequences from plasmid pKH1

1. Growth of cells:

Cells were grown in a 10 liter volume in a Chemap fermenter in 2% medium. Fermentation temperature was 32°C, the pH was 6.8, and air was provided at 1 vvm. Plasmid selection was provided by 50 µg/ml ampicillin. Typical cell yield (wet weight) is 30 g/l.

2. Cell lysis:

50 g, wet cell weight, of *E. coli* containing the recombinant HTLV-III envelope fusion protein were resuspended in a final volume of 100 ml in 50 mM Tris-Cl pH 8.0, 5 mM KEDTA, 5 mM dithiothreitol (DTT), 15 mM β-mercaptoethanol, 0.5% Triton X-100 and 5 mM PMSF. 300 mg lysozyme was added and the suspension incubated for 30 min at room temperature.

This material was lysed using a BEAD-BEATER™ (Biospec Products) containing an equal volume of 0.1-0.15 μm glass beads. The lysis was done for 6 min at room temperature in 1 min intervals. The liquid was separated from the beads and centrifuged for 2.5 hr at 20,000 xg. The supernatant was removed and the pellet dissolved in 100 ml 8 M urea, 20 mM Tris-Cl pH 8.0, 5 mM DTT, 15 mM β-mercaptoethanol, 5 mM PMSF, and 1 mM KEDTA. The pellet was solubilized using a polytron homogenizer (Beckman, Berkeley, CA) and centrifuged at 20,000 xg for 2 hr.

3. DEAE chromatography:

Supernatant was loaded onto a 550 ml column (5 cm x 28 cm) packed with DEAE Fast Flow Sepharose (Pharmacia) equilibrated in 8 M urea, 20 mM Tris-Cl pH 8.0, 15 mM β-mercaptoethanol, and 1 mM KEDTA at room temperature. The column was washed with 1.5 liters equilibration buffer, and the protein eluted with a 5.0 liter linear gradient from 0-0.8 M NaCl in equilibration buffer. The HTLV-III protein eluted at 0.2 M NaCl and was assayed using SDS-polyacrylamide electrophoresis and following the protein at approximately 70 kD.

The fractions containing the HTLV-III protein were pooled and the protein concentrated to 10 ml using a stressed cell positive pressure concentrator (Amicon) fitted with a 10,000 MW cut-off membrane (YM-10, Amicon). The concentrate was loaded onto a 500 ml column (2.5 cm x 100 cm) packed with superfine sephacryl S-300 (Pharmacia) equilibrated in 8 M urea, 20 mM Tris-Cl, pH 8.0, 15 mM β-mercaptoethanol, and 1 mM KEDTA. The column was eluted with equilibration buffer at room temperature. A flow rate of 0.5 ml/min was maintained. The HTLV-III protein eluted at approximately 40% of the column volume.

4. SDS-polyacrylamide electrophoresis:

The fractions containing KH1 were pooled and the protein concentrated using a stressed cell positive pressure concentrator fitted with a 10,000 MW cutoff membrane. 2 mg of protein was mixed with loading buffers and electrophoresed through a preparative SDS-polyacrylamide gel (40 cm x 20 cm x 4 mm) as described by M.W. Hunkapiller, E. Lujan, F. Ostrander, and L.E. Hood, Methods in Enzymology 91:227-236 (1983). The 70 kD HTLV-III protein was visualized with Coomassie blue stain and eluted from the gel as described. The protein can be removed from the SDS by precipitation with acetone (Dynan, W.J., Jendrisak, J.J., Hager, D.A. and Burgess, R.R. [1981] J. Biol. Chem. 256:5860-5865).

Table 1

```
5' GATCAAGCTTCTGCAGTCGACGCATGCGGATCCGGTACCCGGGAGCTCG 3'
       TTCGAAGACGTCAGCTGCGTACGCCTAGGCCATGGGCCCTCGAGCTTAA
```

Table 2

```
5'      CGGTACCAGCCCGCCTAATGAGCGGGCTTTTTTTTGACGT    3'
   TGCAGCCATGGTCGGGCGGATTACTCGCCCGAAAAAAAAC
```

Table 3

```
   MluI      EcoRV  ClaI BamHI  SalI HindIII SmaI
CGAACGCGTGGCCGATATCATCGATGGATCCGTCGACAAGCTTCCCGGGAGCT
GCTTGCGCACCGGCTATAGTAGCTACCTAGGCAGCTGTTCGAAGGGCCC
```

Table 4

5' AATTCCCTGTGTGGAAGGAAGCA
TTAAGGGACACACCTTCCTTCGT

ACCACCACTCTATTTTGTGCATCAGATGCTAAAGCATATGATACAGAGGTACAT
TGGTGGTGAGATAAAACACGTAGTCTACGATTTCGTATACTATGTCTCCATGTA

AATGTTTGGGCCACACATGCCTGTGTACCCACAGACCCCAACCCACAAGAAGTA
TTACAAACCCGGTGTGTACGGACACATGGGTGTCTGGGGTTGGGTGTTCTTCAT

GTATTGGTAAATGTGACAGAAAATTTTAACATGTGGAAAAATGACATGGTAGAA
CATAACCATTTACACTGTCTTTTAAAATTGTACACCTTTTTACTGTACCATCTT

CAGATGCATGAGGATATAATCAGTTTATGGGATCAAAGCCTAAAGCCATGTGTA
GTCTACGTACTCCTATATTAGTCAAATACCCTAGTTTCGGATTTCGGTACACAT

AAATTAACCCCACTCTGTGTTAGTTTAAAGTGCACTGATTTGAAGAATGATACT
TTTAATTGGGGTGAGACACAATCAAATTTCACGTGACTAAACTTCTTACTATGA

AATACCAATAGTAGTAGCGGGAGAATGATAATGGAGAAAGGAGAGATAAAAAAC
TTATGGTTATCATCATCGCCCTCTTACTATTACCTCTTTCCTCTCTATTTTTTG

TGCTCTTTCAATATCAGCACAAGCATAAGAGGTAAGGTGCAGAAAGAATATGCA
ACGAGAAAGTTATAGTCGTGTTCGTATTCTCCATTCCACGTCTTTCTTATACGT

TTTTTTTATAAACTTGATATAATACCAATAGATAATGATACTACCAGCTATACG
AAAAAAATATTTGAACTATATTATGGTTATCTATTACTATGATGGTCGATATGC

TTGACAAGTTGTAACACCTCAGTCATTACACAGGCCTGTCCAAAGGTATCCTTT
AACTGTTCAACATTGTGGAGTCAGTAATGTGTCCGGACAGGTTTCCATAGGAAA

GAGCCAATTCCCATACATTATTGTGCCCCGGCTGGTTTTGCGATTCTAAAATGT
CTCGGTTAAGGGTATGTAATAACACGGGGCCGACCAAAACGCTAAGATTTTACA

AATAATAAGACGTTCAATGGAACAGGACCATGTACAAATGTCAGCACAGTACAA
TTATTATTCTGCAAGTTACCTTGTCCTGGTACATGTTTACAGTCGTGTCATGTT

TGTACACATGGAATTAGGCCAGTAGTATCAACTCAACTGCTGTTAAATGGCAGT
ACATGTGTACCTTAATCCGGTCATCATAGTTGAGTTGACGACAATTTACCGTCA

CTAGCAGAAGAAGAGGTAGTAATTAGATCTGCCAATTTCACAGACAATGCTAAA
GATCGTCTTCTTCTCCATCATTAATCTAGACGGTTAAAGTGTCTGTTACGATTT

ACCATAATAGTACAGCTGAACCAATCTGTAGAAATTAATTGTACAAGACCCAAC
TGGTATTATCATGTCGACTTGGTTAGACATCTTTAATTAACATGTTCTGGGTTG

AACAATACAAGAAAAAGTATCCGTATCCAGAGAGGACCAGGGAGAGCATTTGTT
TTGTTATGTTCTTTTTCATAGGCATAGGTCTCTCCTAATCCCTCTCGTAAACAA

Table 4 (cont.)

ACAATAGGAAAAATAGGAAATATGAGACAAGCACATTGTAACATTAGTAGAGCA
TGTTATCCTTTTTATCCTTTATACTCTGTTCGTGTAACATTGTAATCATCTCGT

AAATGGAATAACACTTTAAAACAGATAGATAGCAAATTAAGAGAACAATTTGGA
TTTACCTTATTGTGAAATTTTGTCTATCTATCGTTTAATTCTCTTGTTAAACCT

AATAATAAAACAATAATCTTTAAGCAGTCCTCAGGAGGGGACCCAGAAATTGTA
TTATTATTTTGTTATTAGAAATTCGTCAGGAGTCCTCCCCTGGGTCTTTAACAT

ACGCACAGTTTTAATTGTGGAGGGGAATTTTTCTACTGTAATTCAACACAACTG
TGCGTGTCAAAATTAACACCTCCCCTTAAAAAGATGACATTAAGTTGTGTTGAC

TTTAATAGTACTTGGTTTAATAGTACTTGGAGTACTAAAGGGTCAAATAACACT
AAATTATCATGAACCAAATTATCATGAACCTCATGATTTCCCAGTTTATTGTGA

GAAGGAAGTGACACAATCACCCTCCCATGCAGAATAAAACAAATTATAAACATG
CTTCCTTCACTGTGTTAGTGGGAGGGTACGTCTTATTTTGTTTAATATTTGTAC

TGGCAGGAAGTAGGAAAAGCAATGTATGCCCCTCCCATCAGTGGACAAATTAGA
ACCGTCCTTCATCCTTTTCGTTACATACGGGGAGGGTAGTCACCTGTTTAATCT

TGTTCATCAAATATTACAGGGCTGCTATTAACAAGAGATGGTGGTAATAGCAAC
ACAAGTAGTTTATAATGTCCCGACGATAATTGTTCTCTACCACCATTATCGTTG

AATGAGTCCGA 3'
TTACTCAGGCTCTAG

Table 5

```
5'   CTGAACCAATCTGTAGAAATTAATTGTACAAGACCCAAC
     GACTTGGTTAGACATCTTTAATTAACATGTTCTGGGTTG

AACAATACAAGAAAAAGTATCCGTATCCAGAGAGGACCAGGGAGAGCATTTGTT
TTGTTATGTTCTTTTTCATAGGCATAGGTCTCTCCTGGTCCCTCTCGTAAACAA

ACAATAGGAAAAATAGGAAATATGAGACAAGCACATTGTAACATTAGTAGAGCA
TGTTATCCTTTTTATCCTTTATACTCTGTTCGTGTAACATTGTAATCATCTCGT

AAATGGAATAACACTTTAAAACAGATAGATAGCAAATTAAGAGAACAATTTGGA
TTTACCTTATTGTGAAATTTTGTCTATCTATCGTTTAATTCTCTTGTTAAACCT

AATAATAAAACAATAATCTTTAAGCAGTCCTCAGGAGGGGACCCAGAAATTGTA
TTATTATTTTGTTATTAGAAATTCGTCAGGAGTCCTCCCCTGGGTCTTTAACAT

ACGCACAGTTTTAATTGTGGAGGGGAATTTTTCTACTGTAATTCAACACAACTG
TGCGTGTCAAAATTAACACCTCCCCTTAAAAAGATGACATTAAGTTGTGTTGAC

TTTAATAGTACTTGGTTTAATAGTACTTGGAGTACTAAAGGGTCAAATAACACT
AAATTATCATGAACCAAATTATCATGAACCTCATGATTTCCCAGTTTATTGTGA

GAAGGAAGTGACACAATCACCCTCCCATGCAGAATAAAACAAATTATAAACATG
CTTCCTTCACTGTGTTAGTGGGAGGGTACGTCTTATTTTGTTTAATATTTGTAC

TGGCAGGAAGTAGGAAAAGCAATGTATGCCCCTCCCATCAGTGGACAAATTAGA
ACCGTCCTTCATCCTTTTCGTTACATACGGGGAGGGTAGTCACCTGTTTAATCT

TGTTCATCAAATATTACAGGGCTGCTATTAACAAGAGATGGTGGTAATAGCAAC
ACAAGTAGTTTATAATGTCCCGACGATAATTGTTCTCTACCACCATTATCGTTG

AATGAGTCCGA          3'
TTACTCAGGCTCTAG
```

Table 6

5' CTGAACCAATCTGTAGAAATTAATTGTACAAGACCCAAC
GACTTGGTTAGACATCTTTAATTAACATGTTCTGGGTTG

AACAATACAAGAAAAAGTATCCGTATCCAGAGAGGACCAGGGAGAGCATTTGTT
TTGTTATGTTCTTTTTCATAGGCATAGGTCTCTCCTGGTCCCTCTCGTAAACAA

ACAATAGGAAAAATAGGAAATATGAGACAAGCACATTGTAACATTAGTAGAGCA
TGTTATCCTTTTTATCCTTTATACTCTGTTCGTGTAACATTGTAATCATCTCGT

AAATGGAATAACACTTTAAAACAGATAGATAGCAAATTAAGAGAACAATTTGGA
TTTACCTTATTGTGAAATTTTGTCTATCTATCGTTTAATTCTCTTGTTAAACCT

AATAATAAAACAATAATCTTTAAGCAGTCCTCAGGAGGGGACCCAGAAATTGTA
TTATTATTTTGTTATTAGAAATTCGTCAGGAGTCCTCCCCTGGGTCTTTAACAT

ACGCACAGTTTTAATTGTGGAGGGGAATTTTTCTACTGTAATTCAACACAACTG
TGCGTGTCAAAATTAACACCTCCCCTTAAAAAGATGACATTAAGTTGTGTTGAC

TTTAATAGTACTTGGTTTAATAGTACTTGGAGTACTAAAGGGTCAAATAACACT
AAATTATCATGAACCAAATTATCATGAACCTCATGATTTCCCAGTTTATTGTGA

GAAGGAAGTGACACAATCACCCTCCCATGCAGAATAAAACAAATTATAAACATG
CTTCCTTCACTGTGTTAGTGGGAGGGTACGTCTTATTTTGTTTAATATTTGTAC

TGGCAGGAAGTAGGAAAAGCAATGTATGCCCCTCCCATCAGTGGACAAATTAGA
ACCGTCCTTCATCCTTTTCGTTACATACGGGGAGGGTAGTCACCTGTTTAATCT

TGTTCATCAAATATTACAGGGCTGCTATTAACAAGAGATGGTGGTAATAGCAAC
ACAAGTAGTTTATAATGTCCCGACGATAATTGTTCTCTACCACCATTATCGTTG

AATGAGTCCGAGATCTTCAGACCTGGAGGAGGAGATATGAGGGACAATTGGAGA
TTACTCAGGCTCTAGAAGTCTGGACCTCCTCCTCTATACTCCCTGTTAACCTCT

AGTGAATTATATAAATATAAAGTAGTAAAAATTGAACCATTAGGAGTAGCACCC
TCACTTAATATATTTATATTTCATCATTTTTAACTTGGTAATCCTCATCGTGGG

ACCAAGGCAAAGAGAAGAGTGGTGCAGAGAGAAAAAAGAGCAGTGGGAATAGGA
TGGTTCCGTTTCTCTTCTCACCACGTCTCTCTTTTTTCTCGTCACCCTTATCCT

GCTTTGTTCCTTGGGTTCTTGGGAGCAGCAGGAAGCACTATGGGCGCAGCGTCA
CGAAACAAGGAACCCAAGAACCCTCGTCGTCCTTCGTGATACCCGCGTCGCAGT

ATGACGCTGACGGTACAGGCCAGACAATTATTGTCTGGTATAGTGCAGCAGCAG
TACTGCGACTGCCATGTCCGGTCTGTTAATAACAGACCATATCACGTCGTCGTC

AACAATTTGCTGAGGGCTATTGAGGCGCAACAGCATCTGTTGCAACTCACAGTC
TTGTTAAACGACTCCCGATAACTCCGCGTTGTCGTAGACAACGTTGAGTGTCAG

TGGGGCATCAAGCAGCTCCAGGCAAGAATCCTGGCTGTGGAAAGATACCTAAAG
ACCCCGTAGTTCGTCGAGGTCCGTTCTTAGGACCGACACCTTTCTATGGATTTC

Table 6 (cont.)

```
GATCAACAGCTCCTGGGGATTTGGGGTTGCTCTGGAAAACTCATTTGCACCACT
CTAGTTGTCGAGGACCCCTAAACCCCAACGAGACCTTTTGAGTAAACGTGGTGA

GCTGTGCCTTGGAATGCTAGTTGGAGTAATAAATCTCTGGAACAGATTTGGAAT
CGACACGGAACCTTACGATCAACCTCATTATTTAGAGACCTTGTCTAAACCTTA

AACATGACCTGGATGGAGTGGGACAGAGAAATTAACAATTACACA         3'
TTGTACTGGACCTACCTCACCCTGTCTCTTTAATTGTTAATGTGTTCGA
```

Table 7

```
                             5'   AATTCCCTGTGTGGAAGGAAGCA
                                  TTAAGGGACACACCTTCCTTCGT

ACCACCACTCTATTTTGTGCATCAGATGCTAAAGCATATGATACAGAGGTACAT
TGGTGGTGAGATAAAACACGTAGTCTACGATTTCGTATACTATGTCTCCATGTA

AATGTTTGGGCCACACATGCCTGTGTACCCACAGACCCCAACCCACAAGAAGTA
TTACAAACCCGGTGTGTACGGACACATGGGTGTCTGGGGTTGGGTGTTCTTCAT

GTATTGGTAAATGTGACAGAAAATTTTAACATGTGGAAAAATGACATGGTAGAA
CATAACCATTTACACTGTCTTTTAAAATTGTACACCTTTTTACTGTACCATCTT

CAGATGCATGAGGATATAATCAGTTTATGGGATCAAAGCCTAAAGCCATGTGTA
GTCTACGTACTCCTATATTAGTCAAATACCCTAGTTTCGGATTTCGGTACACAT

AAATTAACCCCACTCTGTGTTAGTTTAAAGTGCACTGATTTGAAGAATGATACT
TTTAATTGGGGTGAGACACAATCAAATTTCACGTGACTAAACTTCTTACTATGA

AATACCAATAGTAGTAGCGGGAGAATGATAATGGAGAAAGGAGAGATAAAAAAC
TTATGGTTATCATCATCGCCCTCTTACTATTACCTCTTTCCTCTCTATTTTTTG

TGCTCTTTCAATATCAGCACAAGCATAAGAGGTAAGGTGCAGAAAGAATATGCA
ACGAGAAAGTTATAGTCGTGTTCGTATTCTCCATTCCACGTCTTTCTTATACGT

TTTTTTTATAAACTTGATATAATACCAATAGATAATGATACTACCAGCTATACG
AAAAAAATATTTGAACTATATTATGGTTATCTATTACTATGATGGTCGATATGC

TTGACAAGTTGTAACACCTCAGTCATTACACAGGCCTGTCCAAAGGTATCCTTT
AACTGTTCAACATTGTGGAGTCAGTAATGTGTCCGGACAGGTTTCCATAGGAAA

GAGCCAATTCCCATACATTATTGTGCCCCGGCTGGTTTTGCGATTCTAAAATGT
CTCGGTTAAGGGTATGTAATAACACGGGGCCGACCAAAACGCTAAGATTTTACA

AATAATAAGACGTTCAATGGAACAGGACCATGTACAAATGTCAGCACAGTACAA
TTATTATTCTGCAAGTTACCTTGTCCTGGTACATGTTTACAGTCGTGTCATGTT

TGTACACATGGAATTAGGCCAGTAGTATCAACTCAACTGCTGTTAAATGGCAGT
ACATGTGTACCTTAATCCGGTCATCATAGTTGAGTTGACGACAATTTACCGTCA

CTAGCAGAAGAAGAGGTAGTAATTAGATCTGCCAATTTCACAGACAATGCTAAA
GATCGTCTTCTTCTCCATCATTAATCTAGACGGTTAAAGTGTCTGTTACGATTT

ACCATAATAGTACAGCTGAACCAATCTGTAGAAATTAATTGTACAAGACCCAAC
TGGTATTATCATGTCGACTTGGTTAGACATCTTTAATTAACATGTTCTGGGTTG

AACAATACAAGAAAAAGTATCCGTATCCAGAGAGGACCAGGGAGAGCATTTGTT
TTGTTATGTTCTTTTTCATAGGCATAGGTCTCTCCTGGTCCCTCTCGTAAACAA

ACAATAGGAAAAATAGGAAATATGAGACAAGCACATTGTAACATTAGTAGAGCA
TGTTATCCTTTTTATCCTTTATACTCTGTTCGTGTAACATTGTAATCATCTCGT
```

Table 7 (cont.)

AAATGGAATAACACTTTAAAACAGATAGATAGCAAATTAAGAGAACAATTTGGA
TTTACCTTATTGTGAAATTTTGTCTATCTATCGTTTAATTCTCTTGTTAAACCT

AATAATAAAACAATAATCTTTAAGCAGTCCTCAGGAGGGGACCCAGAAATTGTA
TTATTATTTTGTTATTAGAAATTCGTCAGGAGTCCTCCCCTGGGTCTTTAACAT

ACGCACAGTTTTAATTGTGGAGGGGAATTTTTCTACTGTAATTCAACACAACTG
TGCGTGTCAAAATTAACACCTCCCCTTAAAAAGATGACATTAAGTTGTGTTGAC

TTTAATAGTACTTGGTTTAATAGTACTTGGAGTACTAAAGGGTCAAATAACACT
AAATTATCATGAACCAAATTATCATGAACCTCATGATTTCCCAGTTTATTGTGA

GAAGGAAGTGACACAATCACCCTCCCATGCAGAATAAAACAAATTATAAACATG
CTTCCTTCACTGTGTTAGTGGGAGGGTACGTCTTATTTTGTTTAATATTTGTAC

TGGCAGGAAGTAGGAAAAGCAATGTATGCCCCTCCCATCAGTGGACAAATTAGA
ACCGTCCTTCATCCTTTTCGTTACATACGGGGAGGGTAGTCACCTGTTTAATCT

TGTTCATCAAATATTACAGGGCTGCTATTAACAAGAGATGGTGGTAATAGCAAC
ACAAGTAGTTTATAATGTCCCGACGATAATTGTTCTCTACCACCATTATCGTTG

AATGAGTCCGAGATCTTCAGACCTGGAGGAGGAGATATGAGGGACAATTGGAGA
TTACTCAGGCTCTAGAAGTCTGGACCTCCTCCTCTATACTCCCTGTTAACCTCT

AGTGAATTATATAAATATAAAGTAGTAAAAATTGAACCATTAGGAGTAGCACCC
TCACTTAATATATTTATATTTCATCATTTTTAACTTGGTAATCCTCATCGTGGG

ACCAAGGCAAAGAGAAGAGTGGTGCAGAGAGAAAAAAGAGCAGTGGGAATAGGA
TGGTTCCGTTTCTCTTCTCACCACGTCTCTCTTTTTTCTCGTCACCCTTATCCT

GCTTTGTTCCTTGGGTTCTTGGGAGCAGCAGGAAGCACTATGGGCGCAGCGTCA
CGAAACAAGGAACCCAAGAACCCTCGTCGTCCTTCGTGATACCCGCGTCGCAGT

ATGACGCTGACGGTACAGGCCAGACAATTATTGTCTGGTATAGTGCAGCAGCAG
TACTGCGACTGCCATGTCCGGTCTGTTAATAACAGACCATATCACGTCGTCGTC

AACAATTTGCTGAGGGCTATTGAGGCGCAACAGCATCTGTTGCAACTCACAGTC
TTGTTAAACGACTCCCGATAACTCCGCGTTGTCGTAGACAACGTTGAGTGTCAG

TGGGGCATCAAGCAGCTCCAGGCAAGAATCCTGGCTGTGGAAAGATACCTAAAG
ACCCCGTAGTTCGTCGAGGTCCGTTCTTAGGACCGACACCTTTCTATGGATTTC

GATCAACAGCTCCTGGGGATTTGGGGTTGCTCTGGAAAACTCATTTGCACCACT
CTAGTTGTCGAGGACCCCTAAACCCCAACGAGACCTTTTGAGTAAACGTGGTGA

GCTGTGCCTTGGAATGCTAGTTGGAGTAATAAATCTCTGGAACAGATTTGGAAT
CGACACGGAACCTTACGATCAACCTCATTATTTAGAGACCTTGTCTAAACCTTA

AACATGACCTGGATGGAGTGGGACAGAGAAATTAACAATTACACA 3'
TTGTACTGGACCTACCTCACCCTGTCTCTTTAATTGTTAATGTGTTCGA

Table 8

Amino acid sequence of fusion protein R10

MetLeuArg

ProValGluThrProThrArgGluIleLysLysLeuAspGlyLeuTrpAlaPhe
SerLeuAspArgGluAsnCysGlyIleAspGlnPheProValTrpLysGluAla
ThrThrThrLeuPheCysAlaSerAspAlaLysAlaTyrAspThrGluValHis
AsnValTrpAlaThrHisAlaCysValProThrAspProAsnProGlnGluVal
ValLeuValAsnValThrGluAsnPheAsnMetTrpLysAsnAspMetValGlu
GlnMetHisGluAspIleIleSerLeuTrpAspGlnSerLeuLysProCysVal
LysLeuThrProLeuCysValSerLeuLysCysThrAspLeuLysAsnAspThr
AsnThrAsnSerSerSerGlyArgMetIleMetGluLysGlyGluIleLysAsn
CysSerPheAsnIleSerThrSerIleArgGlyLysValGlnLysGluTyrAla
PhePheTyrLysLeuAspIleIleProIleAspAsnAspThrThrSerTyrThr
LeuThrSerCysAsnThrSerValIleThrGlnAlaCysProLysValSerPhe
GluProIleProIleHisTyrCysAlaProAlaGlyPheAlaIleLeuLysCys
AsnAsnLysThrPheAsnGlyThrGlyProCysThrAsnValSerThrValGln
CysThrHisGlyIleArgProValValSerThrGlnLeuLeuLeuAsnGlySer
LeuAlaGluGluGluValValIleArgSerAlaAsnPheThrAspAsnAlaLys
ThrIleIleValGlnLeuAsnGlnSerValGluIleAsnCysThrArgProAsn
AsnAsnThrArgLysSerIleArgIleGlnArgGlyProGlyArgAlaPheVal
ThrIleGlyLysIleGlyAsnMetArgGlnAlaHisCysAsnIleSerArgAla
LysTrpAsnAsnThrLeuLysGlnIleAspSerLysLeuArgGluGlnPheGly
AsnAsnLysThrIleIlePheLysGlnSerSerGlyGlyAspProGluIleVal
ThrHisSerPheAsnCysGlyGlyGluPhePheTyrCysAsnSerThrGlnLeu
PheAsnSerThrTrpPheAsnSerThrTrpSerThrLysGlySerAsnAsnThr
GluGlySerAspThrIleThrLeuProCysArgIleLysGlnIleIleAsnMet
TrpGlnGluValGlyLysAlaMetTyrAlaProProIleSerGlyGlnIleArg
CysSerSerAsnIleThrGlyLeuLeuLeuThrArgAspGlyGlyAsnSerAsn

Table 8 (cont.)

AsnGluSerGluIleHisArgSerValMetLeuTyrThrThrProAsnThrTrp
ValAspAspIleThrValValThrHisValAlaGlnAspCysAsnHisAlaSer
ValAspTrpGlnValValAlaAsnGlyAspValSerValGluLeuArgAspAla
AspGlnGlnValValAlaThrGlyGlnGlyThrSerGlyThrLeuGlnValVal
AsnProHisLeuTrpGlnProGlyGluGlyTyrLeuTyrGluLeuCysValThr
AlaLysSerGlnThrGluCysAspIleTyrProLeuArgValGlyIleArgSer
ValAlaValLysGlyGluGlnPheLeuIleAsnHisLysProPheTyrPheThr
GlyPheGlyArgHisGluAspAlaAspLeuArgGlyLysGlyPheAspAsnVal
LeuMetValHisAspHisAlaLeuMetAspTrpIleGlyAlaAsnSerTyrArg
ThrSerHisTyrProTyrAlaGluGluMetLeuAspTrpAlaAspGluHisGly
IleValValIleAspGluThrAlaAlaValGlyPheAsnLeuSerLeuGlyIle
GlyPheGluAlaGlyAsnLysProLysGluLeuTyrSerGluGluAlaValAsn
GlyGluThrGlnGlnAlaHisLeuGlnAlaIleLysGluLeuIleAlaArgAsp
LysAsnHisProSerValValMetTrpSerIleAlaAsnGluProAspThrArg
ProGlnGlyAlaArgGluTyrPheAlaProLeuAlaGluAlaThrArgLysLeu
AspProThrArgProIleThrCysValAsnValMetPheCysAspAlaHisThr
AspThrIleSerAspLeuPheAspValLeuCysLeuAsnArgTyrTyrGlyTrp
TyrValGlnSerGlyAspLeuGluThrAlaGluLysValLeuGluLysGluLeu
LeuAlaTrpGlnGluLysLeuHisGlnProIleIleIleThrGluTyrGlyVal
AspThrLeuAlaGlyLeuHisSerMetTyrThrAspMetTrpSerGluGluTyr
GlnCysAlaTrpLeuAspMetTyrHisArgValPheAspArgValSerAlaVal
ValGlyGluGlnValTrpAsnPheAlaAspPheAlaThrSerGlnGlyIleLeu
ArgValGlyGlyAsnLysLysGlyIlePheThrArgAspArgLysProLysSer
AlaAlaPheLeuLeuGlnLysArgTrpThrGlyMetAsnPheGlyGluLysPro
GlnGlnGlyGlyLysGln

Table 8A

Nucleotide sequence encoding fusion protein R10

ATGTTACGT
TACAATGCA

CCTGTAGAAACCCCAACCCGTGAAATCAAAAAACTCGACGGCCTGTGGGCATTC
GGACATCTTTGGGGTTGGGCACTTTAGTTTTTTGAGCTGCCGGACACCCGTAAG

AGTCTGGATCGCGAAAACTGTGGAATTGATCAATTCCCTGTGTGGAAGGAAGCA
TCAGACCTAGCGCTTTTGACACCTTAACTAGTTAAGGGACACACCTTCCTTCGT

ACCACCACTCTATTTTGTGCATCAGATGCTAAAGCATATGATACAGAGGTACAT
TGGTGGTGAGATAAAACACGTAGTCTACGATTTCGTATACTATGTCTCCATGTA

AATGTTTGGGCCACACATGCCTGTGTACCCACAGACCCCAACCCACAAGAAGTA
TTACAAACCCGGTGTGTACGGACACATGGGTGTCTGGGGTTGGGTGTTCTTCAT

GTATTGGTAAATGTGACAGAAAATTTTAACATGTGGAAAAATGACATGGTAGAA
CATAACCATTTACACTGTCTTTTAAAATTGTACACCTTTTTACTGTACCATCTT

CAGATGCATGAGGATATAATCAGTTTATGGGATCAAAGCCTAAAGCCATGTGTA
GTCTACGTACTCCTATATTAGTCAAATACCCTAGTTTCGGATTTCGGTACACAT

AAATTAACCCCACTCTGTGTTAGTTTAAAGTGCACTGATTTGAAGAATGATACT
TTTAATTGGGGTGAGACACAATCAAATTTCACGTGACTAAACTTCTTACTATGA

AATACCAATAGTAGTAGCGGGAGAATGATAATGGAGAAAGGAGAGATAAAAAAC
TTATGGTTATCATCATCGCCCTCTTACTATTACCTCTTTCCTCTCTATTTTTTG

TGCTCTTTCAATATCAGCACAAGCATAAGAGGTAAGGTGCAGAAAGAATATGCA
ACGAGAAAGTTATAGTCGTGTTCGTATTCTCCATTCCACGTCTTTCTTATACGT

TTTTTTTATAAACTTGATATAATACCAATAGATAATGATACTACCAGCTATACG
AAAAAAATATTTGAACTATATTATGGTTATCTATTACTATGATGGTCGATATGC

TTGACAAGTTGTAACACCTCAGTCATTACACAGGCCTGTCCAAAGGTATCCTTT
AACTGTTCAACATTGTGGAGTCAGTAATGTGTCCGGACAGGTTTCCATAGGAAA

GAGCCAATTCCCATACATTATTGTGCCCCGGCTGGTTTTGCGATTCTAAAATGT
CTCGGTTAAGGGTATGTAATAACACGGGGCCGACCAAAACGCTAAGATTTTACA

AATAATAAGACGTTCAATGGAACAGGACCATGTACAAATGTCAGCACAGTACAA
TTATTATTCTGCAAGTTACCTTGTCCTGGTACATGTTTACAGTCGTGTCATGTT

TGTACACATGGAATTAGGCCAGTAGTATCAACTCAACTGCTGTTAAATGGCAGT
ACATGTGTACCTTAATCCGGTCATCATAGTTGAGTTGACGACAATTTACCGTCA

CTAGCAGAAGAAGAGGTAGTAATTAGATCTGCCAATTTCACAGACAATGCTAAA
GATCGTCTTCTTCTCCATCATTAATCTAGACGGTTAAAGTGTCTGTTACGATTT

ACCATAATAGTACAGCTGAACCAATCTGTAGAAATTAATTGTACAAGACCCAAC
TGGTATTATCATGTCGACTTGGTTAGACATCTTTAATTAACATGTTCTGGGTTG

Table 8A (cont.)

AACAATACAAGAAAAAGTATCCGTATCCAGAGAGGACCAGGGAGAGCATTTGTT
TTGTTATGTTCTTTTTCATAGGCATAGGTCTCTCCTAATCCCTCTCGTAAACAA

ACAATAGGAAAAATAGGAAATATGAGACAAGCACATTGTAACATTAGTAGAGCA
TGTTATCCTTTTTATCCTTTATACTCTGTTCGTGTAACATTGTAATCATCTCGT

AAATGGAATAACACTTTAAAACAGATAGATAGCAAATTAAGAGAACAATTTGGA
TTTACCTTATTGTGAAATTTTGTCTATCTATCGTTTAATTCTCTTGTTAAACCT

AATAATAAAACAATAATCTTTAAGCAGTCCTCAGGAGGGGACCCAGAAATTGTA
TTATTATTTTGTTATTAGAAATTCGTCAGGAGTCCTCCCCTGGGTCTTTAACAT

ACGCACAGTTTTAATTGTGGAGGGGAATTTTTCTACTGTAATTCAACACAACTG
TGCGTGTCAAAATTAACACCTCCCCTTAAAAAGATGACATTAAGTTGTGTTGAC

TTTAATAGTACTTGGTTTAATAGTACTTGGAGTACTAAAGGGTCAAATAACACT
AAATTATCATGAACCAAATTATCATGAACCTCATGATTTCCCAGTTTATTGTGA

GAAGGAAGTGACACAATCACCCTCCCATGCAGAATAAAACAAATTATAAACATG
CTTCCTTCACTGTGTTAGTGGGAGGGTACGTCTTATTTTGTTTAATATTTGTAC

TGGCAGGAAGTAGGAAAAGCAATGTATGCCCCTCCCATCAGTGGACAAATTAGA
ACCGTCCTTCATCCTTTTCGTTACATACGGGGAGGGTAGTCACCTGTTTAATCT

TGTTCATCAAATATTACAGGGCTGCTATTAACAAGAGATGGTGGTAATAGCAAC
ACAAGTAGTTTATAATGTCCCGACGATAATTGTTCTCTACCACCATTATCGTTG

AATGAGTCCGAGATCCATCGCAGCGTAATGCTCTACACCACGCCGAACACCTGG
TTACTCAGGCTCTAGGTAGCGTCGCATTACGAGATGTGGTGCGGCTTGTGGACC

GTGGACGATATCACCGTGGTGACGCATGTCGCGCAAGACTGTAACCACGCGTCT
CACCTGCTATAGTGGCACCACTGCGTACAGCGCGTTCTGACATTGGTGCGCAGA

GTTGACTGGCAGGTGGTGGCCAATGGTGATGTCAGCGTTGAACTGCGTGATGCG
CAACTGACCGTCCACCACCGGTTACCACTACAGTCGCAACTTGACGCACTACGC

GATCAACAGGTGGTTGCAACTGGACAAGGCACTAGCGGGACTTTGCAAGTGGTG
CTAGTTGTCCACCAACGTTGACCTGTTCCGTGATCGCCCTGAAACGTTCACCAC

AATCCGCACCTCTGGCAACCGGGTGAAGGTTATCTCTATGAACTGTGCGTCACA
TTAGGCGTGGAGACCGTTGGCCCACTTCCAATAGAGATACTTGACACGCAGTGT

GCCAAAAGCCAGACAGAGTGTGATATCTACCCGCTTCGCGTCGGCATCCGGTCA
CGGTTTTCGGTCTGTCTCACACTATAGATGGGCGAAGCGCAGCCGTAGGCCAGT

GTGGCAGTGAAGGGCGAACAGTTCCTGATTAACCACAAACCGTTCTACTTTACT
CACCGTCACTTCCCGCTTGTCAAGGACTAATTGGTGTTTGGCAAGATGAAATGA

GGCTTTGGTCGTCATGAAGATGCGGACTTGCGTGGCAAAGGATTCGATAACGTG
CCGAAACCAGCAGCACTTCTACGCCTGAACGCACCGTTTCCTAAGCTATTGCAC

CTGATGGTGCACGACCACGCATTAATGGACTGGATTGGGGCCAACTCCTACCGT
GACTACCACGTGCTGGTGCGTAATTACCTGACCTAACCCCGGTTGAGGATGGCA

Table 8A (cont.)

ACCTCGCATTACCCTTACGCTGAAGAGATGCTCGACTGGGCAGATGAACATGGC
TGGAGCGTAATGGGAATGCGACTTCTCTACGAGCTGACCCGTCTACTTGTACCG

ATCGTGGTGATTGATGAAACTGCTGCTGTCGGCTTTAACCTCTCTTTAGGCATT
TAGCACCACTAACTACTTTGACGACGACAGCCGAAATTGGAGAGAAATCCGTAA

GGTTTCGAAGCGGGCAACAAGCCGAAAGAACTGTACAGCGAAGAGGCAGTCAAC
CCAAAGCTTCGCCCGTTGTTCGGCTTTCTTGACATGTCGCTTCTCCGTCAGTTG

GGGGAAACTCAGCAAGCGCACTTACAGGCGATTAAAGAGCTGATAGCGCGTGAC
CCCCTTTGAGTCGTTCGCGTGAATGTCCGCTAATTTCTCGACTATCGCGCACTG

AAAAACCACCCAAGCGTGGTGATGTGGAGTATTGCCAACGAACCGGATACCCGT
TTTTTGGTGGGTTCGCACCACTACACCTCATAACGGTTGCTTGGCCTATGGGCA

CCGCAAGGTGCACGGGAATATTTCGCGCCACTGGCGGAAGCAACGCGTAAACTC
GGCGTTCCACGTGCCCTTATAAAGCGCGGTGACCGCCTTCGTTGCGCATTTGAG

GACCCGACGCGTCCGATCACCTGCGTCAATGTAATGTTCTGCGACGCTCACACC
CTGGGCTGCGCAGGCTAGTGGACGCAGTTACATTACAAGACGCTGCGAGTGTGG

GATACCATCAGCGATCTCTTTGATGTGCTGTGCCTGAACCGTTATTACGGATGG
CTATGGTAGTCGCTAGAGAAACTACACGACACGGACTTGGCAATAATGCCTACC

TATGTCCAAAGCGGCGATTTGGAAACGGCAGAGAAGGTACTGGAAAAAGAACTT
ATACAGGTTTCGCCGCTAAACCTTTGCCGTCTCTTCCATGACCTTTTTCTTGAA

CTGGCCTGGCAGGAGAAACTGCATCAGCCGATTATCATCACCGAATACGGCGTG
GACCGGACCGTCCTCTTTGACGTAGTCGGCTAATAGTAGTGGCTTATGCCGCAC

GATACGTTAGCCGGGCTGCACTCAATGTACACCGACATGTGGAGTGAAGAGTAT
CTATGCAATCGGCCCGACGTGAGTTACATGTGGCTGTACACCTCACTTCTCATA

CAGTGTGCATGGCTGGATATGTATCACCGCGTCTTTGATCGCGTCAGCGCCGTC
GTCACACGTACCGACCTATACATAGTGGCGCAGAAACTAGCGCAGTCGCGGCAG

GTCGGTGAACAGGTATGGAATTTCGCCGATTTTGCGACCTCGCAAGGCATATTG
CAGCCACTTGTCCATACCTTAAAGCGGCTAAAACGCTGGAGCGTTCCGTATAAC

CGCGTTGGCGGTAACAAGAAAGGGATCTTCACTCGCGACCGCAAACCGAAGTCG
GCGCAACCGCCATTGTTCTTTCCCTAGAAGTGAGCGCTGGCGTTTGGCTTCAGC

GCGGCTTTTCTGCTGCAAAAACGCTGGACTGGCATGAACTTCGGTGAAAAACCG
CGCCGAAAAGACGACGTTTTTGCGACCTGACCGTACTTGAAGCCACTTTTTGGC

CAGCAGGGAGGCAAACAA
GTCGTCCCTCCGTTTGTT

Table 9

Amino acid sequence of fusion protein PB1

MetLeuArg

ProValGluThrProThrArgGluIleLysLysLeuAspGlyLeuTrpAlaPhe
SerLeuAspArgGluArgValAlaAspLeuAsnGlnSerValGluIleAsnCys
ThrArgProAsnAsnAsnThrArgLysSerIleArgIleGlnArgGlyProGly
ArgAlaPheValThrIleGlyLysIleGlyAsnMetArgGlnAlaHisCysAsn
IleSerArgAlaLysTrpAsnAsnThrLeuLysGlnIleAspSerLysLeuArg
GluGlnPheGlyAsnAsnLysThrIleIlePheLysGlnSerSerGlyGlyAsp
ProGluIleValThrHisSerPheAsnCysGlyGlyGluPhePheTyrCysAsn
SerThrGlnLeuPheAsnSerThrTrpPheAsnSerThrTrpSerThrLysGly
SerAsnAsnThrGluGlySerAspThrIleThrLeuProCysArgIleLysGln
IleIleAsnMetTrpGlnGluValGlyLysAlaMetTyrAlaProProIleSer
GlyGlnIleArgCysSerSerAsnIleThrGlyLeuLeuLeuThrArgAspGly
GlyAsnSerAsnAsnGluSerGluIleArgArgGlnAlaSerArgGluLeuGlu
PheLeuLysThrLysGlyProArgAspThrProIlePheIleGly

Table 9A

Nucleotide sequence encoding fusion protein PB1

ATGTTACGTCCTGTAGAAACCCCAACCCGTGAAATCAAAAAACTCGACGGCCTG
TACAATGCAGGACATCTTTGGGGTTGGGCACTTTAGTTTTTGAGCTGCCGGAC

TGGGCATTCAGTCTGGATCGCGAACGCGTGGCCGATCTGAACCAATCTGTAGAA
ACCCGTAAGTCAGACCTAGCGCTTGCGCACCGGCTAGACTTGGTTAGACATCTT

ATTAATTGTACAAGACCCAACAACAATACAAGAAAAAGTATCCGTATCCAGAGA
TAATTAACATGTTCTGGGTTGTTGTTATGTTCTTTTTCATAGGCATAGGTCTCT

GGACCAGGGAGAGCATTTGTTACAATAGGAAAAATAGGAAATATGAGACAAGCA
CCTAATCCCTCTCGTAAACAATGTTATCCTTTTTATCCTTTATACTCTGTTCGT

CATTGTAACATTAGTAGAGCAAAATGGAATAACACTTTAAAACAGATAGATAGC
GTAACATTGTAATCATCTCGTTTTACCTTATTGTGAAATTTTGTCTATCTATCG

AAATTAAGAGAACAATTTGGAAATAATAAAACAATAATCTTTAAGCAGTCCTCA
TTTAATTCTCTTGTTAAACCTTTATTATTTTGTTATTAGAAATTCGTCAGGAGT

GGAGGGGACCCAGAAATTGTAACGCACAGTTTTAATTGTGGAGGGGAATTTTTC
CCTCCCCTGGGTCTTTAACATTGCGTGTCAAAATTAACACCTCCCCTTAAAAAG

TACTGTAATTCAACACAACTGTTTAATAGTACTTGGTTTAATAGTACTTGGAGT
ATGACATTAAGTTGTGTTGACAAATTATCATGAACCAAATTATCATGAACCTCA

ACTAAAGGGTCAAATAACACTGAAGGAAGTGACACAATCACCCTCCCATGCAGA
TGATTTCCCAGTTTATTGTGACTTCCTTCACTGTGTTAGTGGGAGGGTACGTCT

ATAAAACAAATTATAAACATGTGGCAGGAAGTAGGAAAAGCAATGTATGCCCCT
TATTTTGTTTAATATTTGTACACCGTCCTTCATCCTTTTCGTTACATACGGGGA

CCCATCAGTGGACAAATTAGATGTTCATCAAATATTACAGGGCTGCTATTAACA
GGGTAGTCACCTGTTTAATCTACAAGTAGTTTATAATGTCCCGACGATAATTGT

AGAGATGGTGGTAATAGCAACAATGAGTCCGAGATCCGTCGACAAGCTTCCCGG
TCTCTACCACCATTATCGTTGTTACTCAGGCTCTAGGCAGCTGTTCGAAGGGCC

GAGCTCGAATTCTTGAAGACGAAAGGGCCTCGTGATACTCCTATTTTTATAGGT
CTCGAGCTTAAGAACTTCTGCTTTCCCGGAGCACTATGCGGATAAAAATATCCA

Table 10

Amino acid sequence of fusion protein 590

MetLeuArgProValGluThr
ProThrArgGluIleLysLysLeuAspGlyLeuTrpAlaPheSerLeuAspArg
GluArgValAlaAspLeuAsnGlnSerValGluIleAsnCysThrArgProAsn
AsnAsnThrArgLysSerIleArgIleGlnArgGlyProGlyArgAlaPheVal
ThrIleGlyLysIleGlyAsnMetArgGlnAlaHisCysAsnIleSerArgAla
LysTrpAsnAsnThrLeuLysGlnIleAspSerLysLeuArgGluGlnPheGly
AsnAsnLysThrIleIlePheLysGlnSerSerGlyGlyAspProGluIleVal
ThrHisSerPheAsnCysGlyGlyGluPhePheTyrCysAsnSerThrGlnLeu
PheAsnSerThrTrpPheAsnSerThrTrpSerThrLysGlySerAsnAsnThr
GluGlySerAspThrIleThrLeuProCysArgIleLysGlnIleIleAsnMet
TrpGlnGluValGlyLysAlaMetTyrAlaProProIleSerGlyGlnIleArg
CysSerSerAsnIleThrGlyLeuLeuLeuThrArgAspGlyGlyAsnSerAsn
AsnGluSerGluIlePheArgProGlyGlyGlyAspMetArgAspAsnTrpArg
SerGluLeuTyrLysTyrLysValValLysIleGluProLeuGlyValAlaPro
ThrLysAlaLysArgArgValValGlnArgGluLysArgAlaValGlyIleGly
AlaLeuPheLeuGlyPheLeuGlyAlaAlaGlySerThrMetGlyAlaAlaSer
MetThrLeuThrValGlnAlaArgGlnLeuLeuSerGlyIleValGlnGlnGln
AsnAsnLeuLeuArgAlaIleGluAlaGlnGlnHisLeuLeuGlnLeuThrVal
TrpGlyIleLysGlnLeuGlnAlaArgIleLeuAlaValGluArgTyrLeuLys
AspGlnGlnLeuLeuGlyIleTrpGlyCysSerGlyLysLeuIleCysThrThr
AlaValProTrpAsnAlaSerTrpSerAsnLysSerLeuGluGlnIleTrpAsn
AsnMetThrTrpMetGluTrpAspArgGluIleAsnAsnTyrThrSerPhePro
IleHisArgSerValMetLeuTyrThrThrProAsnThrTrpValAspAspIle
ThrValValThrHisValAlaGlnAspCysAsnHisAlaSerValAspTrpGln
ValValAlaAsnGlyAspValSerValGluLeuArgAspAlaAspGlnGlnVal

Table 10 (cont.)

ValAlaThrGlyGlnGlyThrSerGlyThrLeuGlnValValAsnProHisLeu
TrpGlnProGlyGluGlyTyrLeuTyrGluLeuCysValThrAlaLysSerGln
ThrGluCysAspIleTyrProLeuArgValGlyIleArgSerValAlaValLys
GlyGluGlnPheLeuIleAsnHisLysProPheTyrPheThrGlyPheGlyArg
HisGluAspAlaAspLeuArgGlyLysGlyPheAspAsnValLeuMetValHis
AspHisAlaLeuMetAspTrpIleGlyAlaAsnSerTyrArgThrSerHisTyr
ProTyrAlaGluGluMetLeuAspTrpAlaAspGluHisGlyIleValValIle
AspGluThrAlaAlaValGlyPheAsnLeuSerLeuGlyIleGlyPheGluAla
GlyAsnLysProLysGluLeuTyrSerGluGluAlaValAsnGlyGluThrGln
GlnAlaHisLeuGlnAlaIleLysGluLeuIleAlaArgAspLysAsnHisPro
SerValValMetTrpSerIleAlaAsnGluProAspThrArgProGlnGlyAla
ArgGluTyrPheAlaProLeuAlaGluAlaThrArgLysLeuAspProThrArg
ProIleThrCysValAsnValMetPheCysAspAlaHisThrAspThrIleSer
AspLeuPheAspValLeuCysLeuAsnArgTyrTyrGlyTrpTyrValGlnSer
GlyAspLeuGluThrAlaGluLysValLeuGluLysGluLeuLeuAlaTrpGln
GluLysLeuHisGlnProIleIleIleThrGluTyrGlyValAspThrLeuAla
GlyLeuHisSerMetTyrThrAspMetTrpSerGluGluTyrGlnCysAlaTrp
LeuAspMetTyrHisArgValPheAspArgValSerAlaValValGlyGluGln
ValTrpAsnPheAlaAspPheAlaThrSerGlnGlyIleLeuArgValGlyGly
AsnLysLysGlyIlePheThrArgAspArgLysProLysSerAlaAlaPheLeu
LeuGlnLysArgTrpThrGlyMetAsnPheGlyGluLysProGlnGlnGlyGly
LysGln

Table 10A

Nucleotide sequence encoding fusion protein 590

ATGTTACGTCCTGTAGAAACC
TACAATGCAGGACATCTTTGG

CCAACCCGTGAAATCAAAAAACTCGACGGCCTGTGGGCATTCAGTCTGGATCGC
GGTTGGGCACTTTAGTTTTTTGAGATGCCGGACACCCGTAAGTCAGACCTAGCG

GAACGCGTGGCCGATCTGAACCAATCTGTAGAAATTAATTGTACAAGACCCAAC
CTTGCGCACCGGCTAGACTTGGTTAGACATCTTTAATTAACATGTTCTGGGTTG

AACAATACAAGAAAAAGTATCCGTATCCAGAGAGGACCAGGGAGAGCATTTGTT
TTGTTATGTTCTTTTTCATAGGCATAGGTCTCTCCTAATCCCTCTCGTAAACAA

ACAATAGGAAAAATAGGAAATATGAGACAAGCACATTGTAACATTAGTAGAGCA
TGTTATCCTTTTTATCCTTTATACTCTGTTCGTGTAACATTGTAATCATCTCGT

AAATGGAATAACACTTTAAAACAGATAGATAGCAAATTAAGAGAACAATTTGGA
TTTACCTTATTGTGAAATTTTGTCTATCTATCGTTTAATTCTCTTGTTAAACCT

AATAATAAAACAATAATCTTTAAGCAGTCCTCAGGAGGGGACCCAGAAATTGTA
TTATTATTTTGTTATTAGAAATTCGTCAGGAGTCCTCCCCTGGGTCTTTAACAT

ACGCACAGTTTTAATTGTGGAGGGGAATTTTTCTACTGTAATTCAACACAACTG
TGCGTGTCAAAATTAACACCTCCCCTTAAAAAGATGACATTAAGTTGTGTTGAC

TTTAATAGTACTTGGTTTAATAGTACTTGGAGTACTAAAGGGTCAAATAACACT
AAATTATCATGAACCAAATTATCATGAACCTCATGATTTCCCAGTTTATTGTGA

GAAGGAAGTGACACAATCACCCTCCCATGCAGAATAAAACAAATTATAAACATG
CTTCCTTCACTGTGTTAGTGGGAGGGTACGTCTTATTTTGTTTAATATTTGTAC

TGGCAGGAAGTAGGAAAAGCAATGTATGCCCCTCCCATCAGTGGACAAATTAGA
ACCGTCCTTCATCCTTTTCGTTACATACGGGGAGGGTAGTCACCTGTTTAATCT

TGTTCATCAAATATTACAGGGCTGCTATTAACAAGAGATGGTGGTAATAGCAAC
ACAAGTAGTTTATAATGTCCCGACGATAATTGTTCTCTACCACCATTATCGTTG

AATGAGTCCGAGATCTTCAGACCTGGAGGAGGAGATATGAGGGACAATTGGAGA
TTACTCAGGCTCTAGAAGTCTGGACCTCCTCCTCTATACTCCCTGTTAACCTCT

AGTGAATTATATAAATATAAAGTAGTAAAAATTGAACCATTAGGAGTAGCACCC
TCACTTAATATATTTATATTTCATCATTTTTAACTTGGTAATCCTCATCGTGGG

ACCAAGGCAAAGAGAAGAGTGGTGCAGAGAGAAAAAAGAGCAGTGGGAATAGGA
TGGTTCCGTTTCTCTTCTCACCACGTCTCTCTTTTTTCTCGTCACCCTTATCCT

Table 10A (cont.)

GCTTTGTTCCTTGGGTTCTTGGGAGCAGCAGGAAGCACTATGGGCGCAGCGTCA
CGAAACAAGGAACCCAAGAACCCTCGTCGTCCTTCGTGATACCCGCGTCGCAGT

ATGACGCTGACGGTACAGGCCAGACAATTATTGTCTGGTATAGTGCAGCAGCAG
TACTGCGACTGCCATGTCCGGTCTGTTAATAACAGACCATATCACGTCGTCGTC

AACAATTTGCTGAGGGCTATTGAGGCGCAACAGCATCTGTTGCAACTCACAGTC
TTGTTAAACGACTCCCGATAACTCCGCGTTGTCGTAGACAACGTTGAGTGTCAG

TGGGGCATCAAGCAGCTCCAGGCAAGAATCCTGGCTGTGGAAAGATACCTAAAG
ACCCCGTAGTTCGTCGAGGTCCGTTCTTAGGACCGACACCTTTCTATGGATTTC

GATCAACAGCTCCTGGGGATTTGGGGTTGCTCTGGAAAACTCATTTGCACCACT
CTAGTTGTCGAGGACCCCTAAACCCCAACGAGACCTTTTGAGTAAACGTGGTGA

GCTGTGCCTTGGAATGCTAGTTGGAGTAATAAATCTCTGGAACAGATTTGGAAT
CGACACGGAACCTTACGATCAACCTCATTATTTAGAGACCTTGTCTAAACCTTA

AACATGACCTGGATGGAGTGGGACAGAGAAATTAACAATTACACAAGCTTCCCG
TTGTACTGGACCTACCTCACCCTGTCTCTTTAATTGTTAATGTGTTCGAAGGGC

ATCCATCGCAGCGTAATGCTCTACACCACGCCGAACACCTGGGTGGACGATATC
TAGGTAGCGTCGCATTACGAGATGTGGTGCGGCTTGTGGACCCACCTGCTATAG

ACCGTGGTGACGCATGTCGCGCAAGACTGTAACCACGCGTCTGTTGACTGGCAG
TGGCACCACTGCGTACAGCGCGTTCTGACATTGGTGCGCAGACAACTGACCGTC

GTGGTGGCCAATGGTGATGTCAGCGTTGAACTGCGTGATGCGGATCAACAGGTG
CACCACCGGTTACCACTACAGTCGCAACTTGACGCACTACGCCTAGTTGTCCAC

GTTGCAACTGGACAAGGCACTAGCGGGACTTTGCAAGTGGTGAATCCGCACCTC
CAACGTTGACCTGTTCCGTGATCGCCCTGAAACGTTCACCACTTAGGCGTGGAG

TGGCAACCGGGTGAAGGTTATCTCTATGAACTGTGCGTCACAGCCAAAAGCCAG
ACCGTTGGCCCACTTCCAATAGAGATACTTGACACGCAGTGTCGGTTTTCGGTC

ACAGAGTGTGATATCTACCCGCTTCGCGTCGGCATCCGGTCAGTGGCAGTGAAG
TGTCTCACACTATAGATGGGCGAAGCGCAGCCGTAGGCCAGTCACCGTCACTTC

GGCGAACAGTTCCTGATTAACCACAAACCGTTCTACTTTACTGGCTTTGGTCGT
CCGCTTGTCAAGGACTAATTGGTGTTTGGCAAGATGAAATGACCGAAACCAGCA

CATGAAGATGCGGACTTGCGTGGCAAAGGATTCGATAACGTGCTGATGGTGCAC
GTACTTCTACGCCTGAACGCACCGTTTCCTAAGCTATTGCACGACTACCACGTG

GACCACGCATTAATGGACTGGATTGGGGCCAACTCCTACCGTACCTCGCATTAC
CTGGTGCGTAATTACCTGACCTAACCCCGGTTGAGGATGGCATGGAGCGTAATG

Table 10A (cont.)

CCTTACGCTGAAGAGATGCTCGACTGGGCAGATGAACATGGCATCGTGGTGATT
GGAATGCGACTTCTCTACGAGCTGACCCGTCTACTTGTACCGTAGCACCACTAA

GATGAAACTGCTGCTGTCGGCTTTAACCTCTCTTTAGGCATTGGTTTCGAAGCG
CTACTTTGACGACGACAGCCGAAATTGGAGAGAAATCCGTAACCAAAGCTTCGC

GGCAACAAGCCGAAAGAACTGTACAGCGAAGAGGCAGTCAACGGGGAAACTCAG
CCGTTGTTCGGCTTTCTTGACATGTCGCTTCTCCGTCAGTTGCCCCTTTGAGTC

CAAGCGCACTTACAGGCGATTAAAGAGCTGATAGCGCGTGACAAAAACCACCCA
GTTCGCGTGAATGTCCGCTAATTTCTCGACTATCGCGCACTGTTTTTGGTGGGT

AGCGTGGTGATGTGGAGTATTGCCAACGAACCGGATACCCGTCCGCAAGGTGCA
TCGCACCACTACACCTCATAACGGTTGCTTGGCCTATGGGCAGGCGTTCCACGT

CGGGAATATTTCGCGCCACTGGCGGAAGCAACGCGTAAACTCGACCCGACGCGT
GCCCTTATAAAGCGCGGTGACCGCCTTCGTTGCGCATTTGAGCTGGGCTGCGCA

CCGATCACCTGCGTCAATGTAATGTTCTGCGACGCTCACACCGATACCATCAGC
GGCTAGTGGACGCAGTTACATTACAAGACGCTGCGAGTGTGGCTATGGTAGTCG

GATCTCTTTGATGTGCTGTGCCTGAACCGTTATTACGGATGGTATGTCCAAAGC
CTAGAGAAACTACACGACACGGACTTGGCAATAATGCCTACCATACAGGTTTCG

GGCGATTTGGAAACGGCAGAGAAGGTACTGGAAAAAGAACTTCTGGCCTGGCAG
CCGCTAAACCTTTGCCGTCTCTTCCATGACCTTTTTCTTGAAGACCGGACCGTC

GAGAAACTGCATCAGCCGATTATCATCACCGAATACGGCGTGGATACGTTAGCC
CTCTTTGACGTAGTCGGCTAATAGTAGTGGCTTATGCCGCACCTATGCAATCGG

GGGCTGCACTCAATGTACACCGACATGTGGAGTGAAGAGTATCAGTGTGCATGG
CCCGACGTGAGTTACATGTGGCTGTACACCTCACTTCTCATAGTCACACGTACC

CTGGATATGTATCACCGCGTCTTTGATCGCGTCAGCGCCGTCGTCGGTGAACAG
GACCTATACATAGTGGCGCAGAAACTAGCGCAGTCGCGGCAGCAGCCACTTGTC

GTATGGAATTTCGCCGATTTTGCGACCTCGCAAGGCATATTGCGCGTTGGCGGT
CATACCTTAAAGCGGCTAAAACGCTGGAGCGTTCCGTATAACGCGCAACCGCCA

AACAAGAAAGGGATCTTCACTCGCGACCGCAAACCGAAGTCGGCGGCTTTTCTG
TTGTTCTTTCCCTAGAAGTGAGCGCTGGCGTTTGGCTTCAGCCGCCGAAAAGAC

CTGCAAAAACGCTGGACTGGCATGAACTTCGGTGAAAAACCGCAGCAGGGAGGC
GACGTTTTTGCGACCTGACCGTACTTGAAGCCACTTTTTGGCGTCGTCCCTCCG

AAACAA
TTTGTT

Table 11
Amino acid sequence of fusion protein KH1

MetLeuArg
ProValGluThrProThrArgGluIleLysLysLeuAspGlyLeuTrpAlaPhe
SerLeuAspArgGluArgGluPheProValTrpLysGluAlaThrThrThrLeu
PheCysAlaSerAspAlaLysAlaTyrAspThrGluValHisAsnValTrpAla
ThrHisAlaCysValProThrAspProAsnProGlnGluValValLeuValAsn
ValThrGluAsnPheAsnMetTrpLysAsnAspMetValGluGlnMetHisGlu
AspIleIleSerLeuTrpAspGlnSerLeuLysProCysValLysLeuThrPro
LeuCysValSerLeuLysCysThrAspLeuLysAsnAspThrAsnThrAsnSer
SerSerGlyArgMetIleMetGluLysGlyGluIleLysAsnCysSerPheAsn
IleSerThrSerIleArgGlyLysValGlnLysGluTyrAlaPhePheTyrLys
LeuAspIleIleProIleAspAsnAspThrThrSerTyrThrLeuThrSerCys
AsnThrSerValIleThrGlnAlaCysProLysValSerPheGluProIlePro
IleHisTyrCysAlaProAlaGlyPheAlaIleLeuLysCysAsnAsnLysThr
PheAsnGlyThrGlyProCysThrAsnValSerThrValGlnCysThrHisGly
IleArgProValValSerThrGlnLeuLeuLeuAsnGlySerLeuAlaGluGlu
GluValValIleArgSerAlaAsnPheThrAspAsnAlaLysThrIleIleVal
GlnLeuAsnGlnSerValGluIleAsnCysThrArgProAsnAsnAsnThrArg
LysSerIleArgIleGlnArgGlyProGlyArgAlaPheValThrIleGlyLys
IleGlyAsnMetArgGlnAlaHisCysAsnIleSerArgAlaLysTrpAsnAsn
ThrLeuLysGlnIleAspSerLysLeuArgGluGlnPheGlyAsnAsnLysThr
IleIlePheLysGlnSerSerGlyGlyAspProGluIleValThrHisSerPhe
AsnCysGlyGlyGluPhePheTyrCysAsnSerThrGlnLeuPheAsnSerThr
TrpPheAsnSerThrTrpSerThrLysGlySerAsnAsnThrGluGlySerAsp
ThrIleThrLeuProCysArgIleLysGlnIleIleAsnMetTrpGlnGluVal

Table 11 (cont.)

GlyLysAlaMetTyrAlaProProIleSerGlyGlnIleArgCysSerSerAsn
IleThrGlyLeuLeuLeuThrArgAspGlyGlyAsnSerAsnAsnGluSerGlu
IlePheArgProGlyGlyGlyAspMetArgAspAsnTrpArgSerGluLeuTyr
LysTyrLysValValLysIleGluProLeuGlyValAlaProThrLysAlaLys
ArgArgValValGlnArgGluLysArgAlaValGlyIleGlyAlaLeuPheLeu
GlyPheLeuGlyAlaAlaGlySerThrMetGlyAlaAlaSerMetThrLeuThr
ValGlnAlaArgGlnLeuLeuSerGlyIleValGlnGlnGlnAsnAsnLeuLeu
ArgAlaIleGluAlaGlnGlnHisLeuLeuGlnLeuThrValTrpGlyIleLys
GlnLeuGlnAlaArgIleLeuAlaValGluArgTyrLeuLysAspGlnGlnLeu
LeuGlyIleTrpGlyCysSerGlyLysLeuIleCysThrThrAlaValProTrp
AsnAlaSerTrpSerAsnLysSerLeuGluGlnIleTrpAsnAsnMetThrTrp
MetGluTrpAspArgGluIleAsnAsnTyrThrSerPheProGlyAlaArgIle
LeuGluAspGluArgAlaSer

## Table 11A

Nucleotide sequence encoding fusion protein KH1

```
                                                 ATGTTACGT
                                                 TACAATGCA

CCTGTAGAAACCCCAACCCGTGAAATCAAAAAACTCGACGGCCTGTGGGCATTC
GGACATCTTTGGGGTTGGGCACTTTAGTTTTTTGAGCTGCCGGACACCCGTAAG

AGTCTGGATCGCGAACGCGAATTCCCTGTGTGGAAGGAAGCAACCACCACTCTA
TCAGACCTAGCGCTTGCGCTTAAGGGACACACCTTCCTTCGTTGGTGGTGAGAT

TTTTGTGCATCAGATGCTAAAGCATATGATACAGAGGTACATAATGTTTGGGCC
AAAACACGTAGTCTACGATTTCGTATACTATGTCTCCATGTATTACAAACCCGG

ACACATGCCTGTGTACCCACAGACCCCAACCCACAAGAAGTAGTATTGGTAAAT
TGTGTACGGACACATGGGTGTCTGGGGTTGGGTGTTCTTCATCATAACCATTTA

GTGACAGAAAATTTTAACATGTGGAAAAATGACATGGTAGAACAGATGCATGAG
CACTGTCTTTTAAAATTGTACACCTTTTTACTGTACCATCTTGTCTACGTACTC

GATATAATCAGTTTATGGGATCAAAGCCTAAAGCCATGTGTAAAATTAACCCCA
CTATATTAGTCAAATACCCTAGTTTCGGATTTCGGTACACATTTTAATTGGGGT

CTCTGTGTTAGTTTAAAGTGCACTGATTTGAAGAATGATACTAATACCAATAGT
GAGACACAATCAAATTTCACGTGACTAAACTTCTTACTATGATTATGGTTATCA

AGTAGCGGGAGAATGATAATGGAGAAAGGAGAGATAAAAAACTGCTCTTTCAAT
TCATCGCCCTCTTACTATTACCTCTTTCCTCTCTATTTTTTGACGAGAAAGTTA

ATCAGCACAAGCATAAGAGGTAAGGTGCAGAAAGAATATGCATTTTTTTATAAA
TAGTCGTGTTCGTATTCTCCATTCCACGTCTTTCTTATACGTAAAAAAATATTT

CTTGATATAATACCAATAGATAATGATACTACCAGCTATACGTTGACAAGTTGT
GAACTATATTATGGTTATCTATTACTATGATGGTCGATATGCAACTGTTCAACA

AACACCTCAGTCATTACACAGGCCTGTCCAAAGGTATCCTTTGAGCCAATTCCC
TTGTGGAGTCAGTAATGTGTCCGGACAGGTTTCCATAGGAAACTCGGTTAAGGG

ATACATTATTGTGCCCCGGCTGGTTTTGCGATTCTAAAATGTAATAATAAGACG
TATGTAATAACACGGGGCCGACCAAAACGCTAAGATTTTACATTATTATTCTGC

TTCAATGGAACAGGACCATGTACAAATGTCAGCACAGTACAATGTACACATGGA
AAGTTACCTTGTCCTGGTACATGTTTACAGTCGTGTCATGTTACATGTGTACCT

ATTAGGCCAGTAGTATCAACTCAACTGCTGTTAAATGGCAGTCTAGCAGAAGAA
TAATCCGGTCATCATAGTTGAGTTGACGACAATTTACCGTCAGATCGTCTTCTT

GAGGTAGTAATTAGATCTGCCAATTTCACAGACAATGCTAAAACCATAATAGTA
CTCCATCATTAATCTAGACGGTTAAAGTGTCTGTTACGATTTTGGTATTATCAT
```

Table 11A (cont.)

CAGCTGAACCAATCTGTAGAAATTAATTGTACAAGACCCAACAACAATACAAGA
GTCGACTTGGTTAGACATCTTTAATTAACATGTTCTGGGTTGTTGTTATGTTCT

AAAAGTATCCGTATCCAGAGAGGACCAGGGAGAGCATTTGTTACAATAGGAAAA
TTTTCATAGGCATAGGTCTCTCCTGGTCCCTCTCGTAAACAATGTTATCCTTTT

ATAGGAAATATGAGACAAGCACATTGTAACATTAGTAGAGCAAAATGGAATAAC
TATCCTTTATACTCTGTTCGTGTAACATTGTAATCATCTCGTTTTACCTTATTG

ACTTTAAAACAGATAGATAGCAAATTAAGAGAACAATTTGGAAATAATAAAACA
TGAAATTTTGTCTATCTATCGTTTAATTCTCTTGTTAAACCTTTATTATTTTGT

ATAATCTTTAAGCAGTCCTCAGGAGGGGACCCAGAAATTGTAACGCACAGTTTT
TATTAGAAATTCGTCAGGAGTCCTCCCCTGGGTCTTTAACATTGCGTGTCAAAA

AATTGTGGAGGGGAATTTTTCTACTGTAATTCAACACAACTGTTTAATAGTACT
TTAACACCTCCCCTTAAAAAGATGACATTAAGTTGTGTTGACAAATTATCATGA

TGGTTTAATAGTACTTGGAGTACTAAAGGGTCAAATAACACTGAAGGAAGTGAC
ACCAAATTATCATGAACCTCATGATTTCCCAGTTTATTGTGACTTCCTTCACTG

ACAATCACCCTCCCATGCAGAATAAAACAAATTATAAACATGTGGCAGGAAGTA
TGTTAGTGGGAGGGTACGTCTTATTTTGTTTAATATTTGTACACCGTCCTTCAT

GGAAAAGCAATGTATGCCCCTCCCATCAGTGGACAAATTAGATGTTCATCAAAT
CCTTTTCGTTACATACGGGGAGGGTAGTCACCTGTTTAATCTACAAGTAGTTTA

ATTACAGGGCTGCTATTAACAAGAGATGGTGGTAATAGCAACAATGAGTCCGAG
TAATGTCCCGACGATAATTGTTCTCTACCACCATTATCGTTGTTACTCAGGCTC

ATCTTCAGACCTGGAGGAGGAGATATGAGGGACAATTGGAGAAGTGAATTATAT
TAGAAGTCTGGACCTCCTCCTCTATACTCCCTGTTAACCTCTTCACTTAATATA

AAATATAAAGTAGTAAAAATTGAACCATTAGGAGTAGCACCCACCAAGGCAAAG
TTTATATTTCATCATTTTTAACTTGGTAATCCTCATCGTGGGTGGTTCCGTTTC

AGAAGAGTGGTGCAGAGAGAAAAAAGAGCAGTGGGAATAGGAGCTTTGTTCCTT
TCTTCTCACCACGTCTCTCTTTTTTCTCGTCACCCTTATCCTCGAAACAAGGAA

GGGTTCTTGGGAGCAGCAGGAAGCACTATGGGCGCAGCGTCAATGACGCTGACG
CCCAAGAACCCTCGTCGTCCTTCGTGATACCCGCGTCGCAGTTACTGCGACTGC

GTACAGGCCAGACAATTATTGTCTGGTATAGTGCAGCAGCAGAACAATTTGCTG
CATGTCCGGTCTGTTAATAACAGACCATATCACGTCGTCGTCTTGTTAAACGAC

AGGGCTATTGAGGCGCAACAGCATCTGTTGCAACTCACAGTCTGGGGCATCAAG
TCCCGATAACTCCGCGTTGTCGTAGACAACGTTGAGTGTCAGACCCCGTAGTTC

CAGCTCCAGGCAAGAATCCTGGCTGTGGAAAGATACCTAAAGGATCAACAGCTC
GTCGAGGTCCGTTCTTAGGACCGACACCTTTCTATGGATTTCCTAGTTGTCGAG

Table 11A (cont.)

CTGGGGATTTGGGGTTGCTCTGGAAAACTCATTTGCACCACTGCTGTGCCTTGG
GACCCCTAAACCCCAACGAGACCTTTTGAGTAAACGTGGTGACGACACGGAACC

AATGCTAGTTGGAGTAATAAATCTCTGGAACAGATTTGGAATAACATGACCTGG
TTACGATCAACCTCATTATTTAGAGACCTTGTCTAAACCTTATTGTACTGGACC

ATGGAGTGGGACAGAGAAATTAACAATTACACAAGCTTCCCGGGAGCTCGAATT
TACCTCACCCTGTCTCTTTAATTGTTAATGTGTTCGAAGGGCCCTCGAGCTTAA

CTTGAAGACGAAAGGGCCTCG
GAACTTCTGCTTTCCCGGAGC

## CLAIMS

1. A recombinant DNA transfer vector comprising DNA having all or part of the following nucleotide sequence or equivalent nucleotide sequences containing bases whose translated region codes for HTLV-III envelope protein fragment denoted R10:

```
                                             ATGTTACGT
                                             TACAATGCA

CCTGTAGAAACCCCAACCCGTGAAATCAAAAAACTCGACGGCCTGTGGGCATTC
GGACATCTTTGGGGTTGGGCACTTTAGTTTTTGAGCTGCCGGACACCCGTAAG

AGTCTGGATCGCGAAAACTGTGGAATTGATCAATTCCCTGTGTGGAAGGAAGCA
TCAGACCTAGCGCTTTTGACACCTTAACTAGTTAAGGGACACACCTTCCTTCGT

ACCACCACTCTATTTTGTGCATCAGATGCTAAAGCATATGATACAGAGGTACAT
TGGTGGTGAGATAAAACACGTAGTCTACGATTTCGTATACTATGTCTCCATGTA

AATGTTTGGGCCACACATGCCTGTGTACCCACAGACCCCAACCCACAAGAAGTA
TTACAAACCCGGTGTGTACGGACACATGGGTGTCTGGGGTTGGGTGTTCTTCAT

GTATTGGTAAATGTGACAGAAAATTTTAACATGTGGAAAAATGACATGGTAGAA
CATAACCATTTACACTGTCTTTTAAAATTGTACACCTTTTTACTGTACCATCTT

CAGATGCATGAGGATATAATCAGTTTATGGGATCAAAGCCTAAAGCCATGTGTA
GTCTACGTACTCCTATATTAGTCAAATACCCTAGTTTCGGATTTCGGTACACAT

AAATTAACCCCACTCTGTGTTAGTTTAAAGTGCACTGATTTGAAGAATGATACT
TTTAATTGGGGTGAGACACAATCAAATTTCACGTGACTAAACTTCTTACTATGA

AATACCAATAGTAGTAGCGGGAGAATGATAATGGAGAAAGGAGAGATAAAAAAC
TTATGGTTATCATCATCGCCCTCTTACTATTACCTCTTTCCTCTCTATTTTTTG

TGCTCTTTCAATATCAGCACAAGCATAAGAGGTAAGGTGCAGAAAGAATATGCA
ACGAGAAAGTTATAGTCGTGTTCGTATTCTCCATTCCACGTCTTTCTTATACGT

TTTTTTTATAAACTTGATATAATACCAATAGATAATGATACTACCAGCTATACG
AAAAAAATATTTGAACTATATTATGGTTATCTATTACTATGATGGTCGATATGC

TTGACAAGTTGTAACACCTCAGTCATTACACAGGCCTGTCCAAAGGTATCCTTT
AACTGTTCAACATTGTGGAGTCAGTAATGTGTCCGGACAGGTTTCCATAGGAAA

GAGCCAATTCCCATACATTATTGTGCCCCGGCTGGTTTTGCGATTCTAAAATGT
CTCGGTTAAGGGTATGTAATAACACGGGGCCGACCAAAACGCTAAGATTTTACA

AATAATAAGACGTTCAATGGAACAGGACCATGTACAAATGTCAGCACAGTACAA
TTATTATTCTGCAAGTTACCTTGTCCTGGTACATGTTTACAGTCGTGTCATGTT
```

34 TGTACACATGGAATTAGGCCAGTAGTATCAACTCAACTGCTGTTAAATGGCAGT
35 ACATGTGTACCTTAATCCGGTCATCATAGTTGAGTTGACGACAATTTACCGTCA

36 CTAGCAGAAGAAGAGGTAGTAATTAGATCTGCCAATTTCACAGACAATGCTAAA
37 GATCGTCTTCTTCTCCATCATTAATCTAGACGGTTAAAGTGTCTGTTACGATTT

38 ACCATAATAGTACAGCTGAACCAATCTGTAGAAATTAATTGTACAAGACCCAAC
39 TGGTATTATCATGTCGACTTGGTTAGACATCTTTAATTAACATGTTCTGGGTTG

40 AACAATACAAGAAAAAGTATCCGTATCCAGAGAGGACCAGGGAGAGCATTTGTT
41 TTGTTATGTTCTTTTTCATAGGCATAGGTCTCTCCTAATCCCTCTCGTAAACAA

42 ACAATAGGAAAAATAGGAAATATGAGACAAGCACATTGTAACATTAGTAGAGCA
43 TGTTATCCTTTTTATCCTTTATACTCTGTTCGTGTAACATTGTAATCATCTCGT

44 AAATGGAATAACACTTTAAAACAGATAGATAGCAAATTAAGAGAACAATTTGGA
45 TTTACCTTATTGTGAAATTTTGTCTATCTATCGTTTAATTCTCTTGTTAAACCT

46 AATAATAAAACAATAATCTTTAAGCAGTCCTCAGGAGGGGACCCAGAAATTGTA
47 TTATTATTTTGTTATTAGAAATTCGTCAGGAGTCCTCCCCTGGGTCTTTAACAT

48 ACGCACAGTTTTAATTGTGGAGGGGAATTTTTCTACTGTAATTCAACACAACTG
49 TGCGTGTCAAAATTAACACCTCCCCTTAAAAAGATGACATTAAGTTGTGTTGAC

50 TTTAATAGTACTTGGTTTAATAGTACTTGGAGTACTAAAGGGTCAAATAACACT
51 AAATTATCATGAACCAAATTATCATGAACCTCATGATTTCCCAGTTTATTGTGA

52 GAAGGAAGTGACACAATCACCCTCCCATGCAGAATAAAACAAATTATAAACATG
53 CTTCCTTCACTGTGTTAGTGGGAGGGTACGTCTTATTTTGTTTAATATTTGTAC

54 TGGCAGGAAGTAGGAAAAGCAATGTATGCCCCTCCCATCAGTGGACAAATTAGA
55 ACCGTCCTTCATCCTTTTCGTTACATACGGGGAGGGTAGTCACCTGTTTAATCT

56 TGTTCATCAAATATTACAGGGCTGCTATTAACAAGAGATGGTGGTAATAGCAAC
57 ACAAGTAGTTTATAATGTCCCGACGATAATTGTTCTCTACCACCATTATCGTTG

58 AATGAGTCCGAGATCCATCGCAGCGTAATGCTCTACACCACGCCGAACACCTGG
59 TTACTCAGGCTCTAGGTAGCGTCGCATTACGAGATGTGGTGCGGCTTGTGGACC

60 GTGGACGATATCACCGTGGTGACGCATGTCGCGCAAGACTGTAACCACGCGTCT
61 CACCTGCTATAGTGGCACCACTGCGTACAGCGCGTTCTGACATTGGTGCGCAGA

62 GTTGACTGGCAGGTGGTGGCCAATGGTGATGTCAGCGTTGAACTGCGTGATGCG
63 CAACTGACCGTCCACCACCGGTTACCACTACAGTCGCAACTTGACGCACTACGC

64 GATCAACAGGTGGTTGCAACTGGACAAGGCACTAGCGGGACTTTGCAAGTGGTG
65 CTAGTTGTCCACCAACGTTGACCTGTTCCGTGATCGCCCTGAAACGTTCACCAC

66 AATCCGCACCTCTGGCAACCGGGTGAAGGTTATCTCTATGAACTGTGCGTCACA
67 TTAGGCGTGGAGACCGTTGGCCCACTTCCAATAGAGATACTTGACACGCAGTGT

68 GCCAAAAGCCAGACAGAGTGTGATATCTACCCGCTTCGCGTCGGCATCCGGTCA
69 CGGTTTTCGGTCTGTCTCACACTATAGATGGGCGAAGCGCAGCCGTAGGCCAGT

70 GTGGCAGTGAAGGGCGAACAGTTCCTGATTAACCACAAACCGTTCTACTTTACT
71 CACCGTCACTTCCCGCTTGTCAAGGACTAATTGGTGTTTGGCAAGATGAAATGA

72 GGCTTTGGTCGTCATGAAGATGCGGACTTGCGTGGCAAAGGATTCGATAACGTG
73 CCGAAACCAGCAGCACTTCTACGCCTGAACGCACCGTTTCCTAAGCTATTGCAC

74 CTGATGGTGCACGACCACGCATTAATGGACTGGATTGGGGCCAACTCCTACCGT
75 GACTACCACGTGCTGGTGCGTAATTACCTGACCTAACCCCGGTTGAGGATGGCA

76 ACCTCGCATTACCCTTACGCTGAAGAGATGCTCGACTGGGCAGATGAACATGGC
77 TGGAGCGTAATGGGAATGCGACTTCTCTACGAGCTGACCCGTCTACTTGTACCG

78 ATCGTGGTGATTGATGAAACTGCTGCTGTCGGCTTTAACCTCTCTTTAGGCATT
79 TAGCACCACTAACTACTTTGACGACGACAGCCGAAATTGGAGAGAAATCCGTAA

80 GGTTTCGAAGCGGGCAACAAGCCGAAAGAACTGTACAGCGAAGAGGCAGTCAAC
81 CCAAAGCTTCGCCCGTTGTTCGGCTTTCTTGACATGTCGCTTCTCCGTCAGTTG

82 GGGGAAACTCAGCAAGCGCACTTACAGGCGATTAAAGAGCTGATAGCGCGTGAC
83 CCCCTTTGAGTCGTTCGCGTGAATGTCCGCTAATTTCTCGACTATCGCGCACTG

84 AAAAACCACCCAAGCGTGGTGATGTGGAGTATTGCCAACGAACCGGATACCCGT
85 TTTTTGGTGGGTTCGCACCACTACACCTCATAACGGTTGCTTGGCCTATGGGCA

86 CCGCAAGGTGCACGGGAATATTTCGCGCCACTGGCGGAAGCAACGCGTAAACTC
87 GGCGTTCCACGTGCCCTTATAAAGCGCGGTGACCGCCTTCGTTGCGCATTTGAG

88 GACCCGACGCGTCCGATCACCTGCGTCAATGTAATGTTCTGCGACGCTCACACC
89 CTGGGCTGCGCAGGCTAGTGGACGCAGTTACATTACAAGACGCTGCGAGTGTGG

90 GATACCATCAGCGATCTCTTTGATGTGCTGTGCCTGAACCGTTATTACGGATGG
91 CTATGGTAGTCGCTAGAGAAACTACACGACACGGACTTGGCAATAATGCCTACC

92 TATGTCCAAAGCGGCGATTTGGAAACGGCAGAGAAGGTACTGGAAAAAGAACTT
93 ATACAGGTTTCGCCGCTAAACCTTTGCCGTCTCTTCCATGACCTTTTTCTTGAA

94 CTGGCCTGGCAGGAGAAACTGCATCAGCCGATTATCATCACCGAATACGGCGTG
95 GACCGGACCGTCCTCTTTGACGTAGTCGGCTAATAGTAGTGGCTTATGCCGCAC

96 GATACGTTAGCCGGGCTGCACTCAATGTACACCGACATGTGGAGTGAAGAGTAT
97 CTATGCAATCGGCCCGACGTGAGTTACATGTGGCTGTACACCTCACTTCTCATA

98 CAGTGTGCATGGCTGGATATGTATCACCGCGTCTTTGATCGCGTCAGCGCCGTC
99 GTCACACGTACCGACCTATACATAGTGGCGCAGAAACTAGCGCAGTCGCGGCAG

100 GTCGGTGAACAGGTATGGAATTTCGCCGATTTTGCGACCTCGCAAGGCATATTG
101 CAGCCACTTGTCCATACCTTAAAGCGGCTAAAACGCTGGAGCGTTCCGTATAAC

102 CGCGTTGGCGGTAACAAGAAAGGGATCTTCACTCGCGACCGCAAACCGAAGTCG
103 GCGCAACCGCCATTGTTCTTTCCCTAGAAGTGAGCGCTGGCGTTTGGCTTCAGC

104 GCGGCTTTTCTGCTGCAAAAACGCTGGACTGGCATGAACTTCGGTGAAAAACCG
105 CGCCGAAAAGACGACGTTTTTGCGACCTGACCGTACTTGAAGCCACTTTTTGGC

106 CAGCAGGGAGGCAAACAA
107 GTCGTCCCTCCGTTTGTT.

2. A recombinant DNA transfer vector comprising DNA having all or part of the following nucleotide sequence or equivalent nucleotide sequences containing bases whose translated region codes for HTLV-III envelope protein fragment denoted PB1:

ATGTTACGTCCTGTAGAAACCCCAACCCGTGAAATCAAAAAACTCGACGGCCTG
TACAATGCAGGACATCTTTGGGGTTGGGCACTTTAGTTTTTTGAGCTGCCGGAC

TGGGCATTCAGTCTGGATCGCGAACGCGTGGCCGATCTGAACCAATCTGTAGAA
ACCCGTAAGTCAGACCTAGCGCTTGCGCACCGGCTAGACTTGGTTAGACATCTT

ATTAATTGTACAAGACCCAACAACAATACAAGAAAAAGTATCCGTATCCAGAGA
TAATTAACATGTTCTGGGTTGTTGTTATGTTCTTTTTCATAGGCATAGGTCTCT

GGACCAGGGAGAGCATTTGTTACAATAGGAAAAATAGGAAATATGAGACAAGCA
CCTAATCCCTCTCGTAAACAATGTTATCCTTTTTATCCTTTATACTCTGTTCGT

CATTGTAACATTAGTAGAGCAAAATGGAATAACACTTTAAAACAGATAGATAGC
GTAACATTGTAATCATCTCGTTTTACCTTATTGTGAAATTTTGTCTATCTATCG

AAATTAAGAGAACAATTTGGAAATAATAAAACAATAATCTTTAAGCAGTCCTCA
TTTAATTCTCTTGTTAAACCTTTATTATTTTGTTATTAGAAATTCGTCAGGAGT

GGAGGGGACCCAGAAATTGTAACGCACAGTTTTAATTGTGGAGGGGAATTTTTC
CCTCCCCTGGGTCTTTAACATTGCGTGTCAAAATTAACACCTCCCCTTAAAAAG

TACTGTAATTCAACACAACTGTTTAATAGTACTTGGTTTAATAGTACTTGGAGT
ATGACATTAAGTTGTGTTGACAAATTATCATGAACCAAATTATCATGAACCTCA

ACTAAAGGGTCAAATAACACTGAAGGAAGTGACACAATCACCCTCCCATGCAGA
TGATTTCCCAGTTTATTGTGACTTCCTTCACTGTGTTAGTGGGAGGGTACGTCT

ATAAAACAAATTATAAACATGTGGCAGGAAGTAGGAAAAGCAATGTATGCCCCT
TATTTTGTTTAATATTTGTACACCGTCCTTCATCCTTTTCGTTACATACGGGGA

CCCATCAGTGGACAAATTAGATGTTCATCAAATATTACAGGGCTGCTATTAACA
GGGTAGTCACCTGTTTAATCTACAAGTAGTTTATAATGTCCCGACGATAATTGT

AGAGATGGTGGTAATAGCAACAATGAGTCCGAGATCCGTCGACAAGCTTCCCGG
TCTCTACCACCATTATCGTTGTTACTCAGGCTCTAGGCAGCTGTTCGAAGGGCC

GAGCTCGAATTCTTGAAGACGAAAGGGCCTCGTGATACTCCTATTTTTATAGGT
CTCGAGCTTAAGAACTTCTGCTTTCCCGGAGCACTATGCGGATAAAAATATCCA.

3. A recombinant DNA transfer vector comprising DNA having all or part of the following nucleotide sequence or equivalent nucleotide sequences containing bases whose translated region codes for HTLV-III envelope protein fragment denoted 590:

```
                                               ATGTTACGTCCTGTAGAAACC
                                               TACAATGCAGGACATCTTTGG

CCAACCCGTGAAATCAAAAAACTCGACGGCCTGTGGGCATTCAGTCTGGATCGC
GGTTGGGCACTTTAGTTTTTGAGATGCCGGACACCCGTAAGTCAGACCTAGCG

GAACGCGTGGCCGATCTGAACCAATCTGTAGAAATTAATTGTACAAGACCCAAC
CTTGCGCACCGGCTAGACTTGGTTAGACATCTTTAATTAACATGTTCTGGGTTG

AACAATACAAGAAAAAGTATCCGTATCCAGAGAGGACCAGGGAGAGCATTTGTT
TTGTTATGTTCTTTTTCATAGGCATAGGTCTCTCCTAATCCCTCTCGTAAACAA

ACAATAGGAAAAATAGGAAATATGAGACAAGCACATTGTAACATTAGTAGAGCA
TGTTATCCTTTTTATCCTTTATACTCTGTTCGTGTAACATTGTAATCATCTCGT

AAATGGAATAACACTTTAAAACAGATAGATAGCAAATTAAGAGAACAATTTGGA
TTTACCTTATTGTGAAATTTTGTCTATCTATCGTTTAATTCTCTTGTTAAACCT

AATAATAAAACAATAATCTTTAAGCAGTCCTCAGGAGGGGACCCAGAAATTGTA
TTATTATTTTGTTATTAGAAATTCGTCAGGAGTCCTCCCCTGGGTCTTTAACAT

ACGCACAGTTTTAATTGTGGAGGGGAATTTTTCTACTGTAATTCAACACAACTG
TGCGTGTCAAAATTAACACCTCCCCTTAAAAAGATGACATTAAGTTGTGTTGAC

TTTAATAGTACTTGGTTTAATAGTACTTGGAGTACTAAAGGGTCAAATAACACT
AAATTATCATGAACCAAATTATCATGAACCTCATGATTTCCCAGTTTATTGTGA

GAAGGAAGTGACACAATCACCCTCCCATGCAGAATAAAACAAATTATAAACATG
CTTCCTTCACTGTGTTAGTGGGAGGGTACGTCTTATTTTGTTTAATATTTGTAC

TGGCAGGAAGTAGGAAAAGCAATGTATGCCCCTCCCATCAGTGGACAAATTAGA
ACCGTCCTTCATCCTTTTCGTTACATACGGGGAGGGTAGTCACCTGTTTAATCT

TGTTCATCAAATATTACAGGGCTGCTATTAACAAGAGATGGTGGTAATAGCAAC
ACAAGTAGTTTATAATGTCCCGACGATAATTGTTCTCTACCACCATTATCGTTG

AATGAGTCCGAGATCTTCAGACCTGGAGGAGGAGATATGAGGGACAATTGGAGA
TTACTCAGGCTCTAGAAGTCTGGACCTCCTCCTCTATACTCCCTGTTAACCTCT

AGTGAATTATATAAATATAAAGTAGTAAAAATTGAACCATTAGGAGTAGCACCC
TCACTTAATATATTTATATTTCATCATTTTTAACTTGGTAATCCTCATCGTGGG

ACCAAGGCAAAGAGAAGAGTGGTGCAGAGAGAAAAAAGAGCAGTGGGAATAGGA
TGGTTCCGTTTCTCTTCTCACCACGTCTCTCTTTTTTCTCGTCACCCTTATCCT
```

36 GCTTTGTTCCTTGGGTTCTTGGGAGCAGCAGGAAGCACTATGGGCGCAGCGTCA
37 CGAAACAAGGAACCCAAGAACCCTCGTCGTCCTTCGTGATACCCGCGTCGCAGT

38 ATGACGCTGACGGTACAGGCCAGACAATTATTGTCTGGTATAGTGCAGCAGCAG
39 TACTGCGACTGCCATGTCCGGTCTGTTAATAACAGACCATATCACGTCGTCGTC

40 AACAATTTGCTGAGGGCTATTGAGGCGCAACAGCATCTGTTGCAACTCACAGTC
41 TTGTTAAACGACTCCCGATAACTCCGCGTTGTCGTAGACAACGTTGAGTGTCAG

42 TGGGGCATCAAGCAGCTCCAGGCAAGAATCCTGGCTGTGGAAAGATACCTAAAG
43 ACCCCGTAGTTCGTCGAGGTCCGTTCTTAGGACCGACACCTTTCTATGGATTTC

44 GATCAACAGCTCCTGGGGATTTGGGGTTGCTCTGGAAAACTCATTTGCACCACT
45 CTAGTTGTCGAGGACCCCTAAACCCCAACGAGACCTTTTGAGTAAACGTGGTGA

46 GCTGTGCCTTGGAATGCTAGTTGGAGTAATAAATCTCTGGAACAGATTTGGAAT
47 CGACACGGAACCTTACGATCAACCTCATTATTTAGAGACCTTGTCTAAACCTTA

48 AACATGACCTGGATGGAGTGGGACAGAGAAATTAACAATTACACAAGCTTCCCG
49 TTGTACTGGACCTACCTCACCCTGTCTCTTTAATTGTTAATGTGTTCGAAGGGC

50 ATCCATCGCAGCGTAATGCTCTACACCACGCCGAACACCTGGGTGGACGATATC
51 TAGGTAGCGTCGCATTACGAGATGTGGTGCGGCTTGTGGACCCACCTGCTATAG

52 ACCGTGGTGACGCATGTCGCGCAAGACTGTAACCACGCGTCTGTTGACTGGCAG
53 TGGCACCACTGCGTACAGCGCGTTCTGACATTGGTGCGCAGACAACTGACCGTC

54 GTGGTGGCCAATGGTGATGTCAGCGTTGAACTGCGTGATGCGGATCAACAGGTG
55 CACCACCGGTTACCACTACAGTCGCAACTTGACGCACTACGCCTAGTTGTCCAC

56 GTTGCAACTGGACAAGGCACTAGCGGGACTTTGCAAGTGGTGAATCCGCACCTC
57 CAACGTTGACCTGTTCCGTGATCGCCCTGAAACGTTCACCACTTAGGCGTGGAG

58 TGGCAACCGGGTGAAGGTTATCTCTATGAACTGTGCGTCACAGCCAAAAGCCAG
59 ACCGTTGGCCCACTTCCAATAGAGATACTTGACACGCAGTGTCGGTTTTCGGTC

60 ACAGAGTGTGATATCTACCCGCTTCGCGTCGGCATCCGGTCAGTGGCAGTGAAG
61 TGTCTCACACTATAGATGGGCGAAGCGCAGCCGTAGGCCAGTCACCGTCACTTC

62 GGCGAACAGTTCCTGATTAACCACAAACCGTTCTACTTTACTGGCTTTGGTCGT
63 CCGCTTGTCAAGGACTAATTGGTGTTTGGCAAGATGAAATGACCGAAACCAGCA

64 CATGAAGATGCGGACTTGCGTGGCAAAGGATTCGATAACGTGCTGATGGTGCAC
65 GCACTTCTACGCCTGAACGCACCGTTTCCTAAGCTATTGCACGACTACCACGTG

66 GACCACGCATTAATGGACTGGATTGGGGCCAACTCCTACCGTACCTCGCATTAC
67 CTGGTGCGTAATTACCTGACCTAACCCCGGTTGAGGATGGCATGGAGCGTAATG

68 CCTTACGCTGAAGAGATGCTCGACTGGGCAGATGAACATGGCATCGTGGTGATT
69 GGAATGCGACTTCTCTACGAGCTGACCCGTCTACTTGTACCGTAGCACCACTAA

70 GATGAAACTGCTGCTGTCGGCTTTAACCTCTCTTTAGGCATTGGTTTCGAAGCG
71 CTACTTTGACGACGACAGCCGAAATTGGAGAGAAATCCGTAACCAAAGCTTCGC

72 GGCAACAAGCCGAAAGAACTGTACAGCGAAGAGGCAGTCAACGGGGAAACTCAG
73 CCGTTGTTCGGCTTTCTTGACATGTCGCTTCTCCGTCAGTTGCCCCTTTGAGTC

74 CAAGCGCACTTACAGGCGATTAAAGAGCTGATAGCGCGTGACAAAAACCACCCA
75 GTTCGCGTGAATGTCCGCTAATTTCTCGACTATCGCGCACTGTTTTTGGTGGGT

76 AGCGTGGTGATGTGGAGTATTGCCAACGAACCGGATACCCGTCCGCAAGGTGCA
77 TCGCACCACTACACCTCATAACGGTTGCTTGGCCTATGGGCAGGCGTTCCACGT

78 CGGGAATATTTCGCGCCACTGGCGGAAGCAACGCGTAAACTCGACCCGACGCGT
79 GCCCTTATAAAGCGCGGTGACCGCCTTCGTTGCGCATTTGAGCTGGGCTGCGCA

80 CCGATCACCTGCGTCAATGTAATGTTCTGCGACGCTCACACCGATACCATCAGC
81 GGCTAGTGGACGCAGTTACATTACAAGACGCTGCGAGTGTGGCTATGGTAGTCG

82 GATCTCTTTGATGTGCTGTGCCTGAACCGTTATTACGGATGGTATGTCCAAAGC
83 CTAGAGAAACTACACGACACGGACTTGGCAATAATGCCTACCATACAGGTTTCG

84 GGCGATTTGGAAACGGCAGAGAAGGTACTGGAAAAAGAACTTCTGGCCTGGCAG
85 CCGCTAAACCTTTGCCGTCTCTTCCATGACCTTTTTCTTGAAGACCGGACCGTC

86 GAGAAACTGCATCAGCCGATTATCATCACCGAATACGGCGTGGATACGTTAGCC
87 CTCTTTGACGTAGTCGGCTAATAGTAGTGGCTTATGCCGCACCTATGCAATCGG

88 GGGCTGCACTCAATGTACACCGACATGTGGAGTGAAGAGTATCAGTGTGCATGG
89 CCCGACGTGAGTTACATGTGGCTGTACACCTCACTTCTCATAGTCACACGTACC

90 CTGGATATGTATCACCGCGTCTTTGATCGCGTCAGCGCCGTCGTCGGTGAACAG
91 GACCTATACATAGTGGCGCAGAAACTAGCGCAGTCGCGGCAGCAGCCACTTGTC

92 GTATGGAATTTCGCCGATTTTGCGACCTCGCAAGGCATATTGCGCGTTGGCGGT
93 CATACCTTAAAGCGGCTAAAACGCTGGAGCGTTCCGTATAACGCGCAACCGCCA

94 AACAAGAAAGGGATCTTCACTCGCGACCGCAAACCGAAGTCGGCGGCTTTTCTG
95 TTGTTCTTTCCCTAGAAGTGAGCGCTGGCGTTTGGCTTCAGCCGCCGAAAAGAC

96 CTGCAAAAACGCTGGACTGGCATGAACTTCGGTGAAAAACCGCAGCAGGGAGGC
97 GACGTTTTTGCGACCTGACCGTACTTGAAGCCACTTTTTGGCGTCGTCCCTCCG

98 AAACAA
99 TTTGTT.

4. A recombinant DNA transfer vector comprising DNA having all or part of the following nucleotide sequence or equivalent nucleotide sequences containing bases whose translated region codes for HTLV-III envelope protein fragment denoted KH1:

```
                                                ATGTTACGT
                                                TACAATGCA

CCTGTAGAAACCCCAACCCGTGAAATCAAAAAACTCGACGGCCTGTGGGCATTC
GGACATCTTTGGGGTTGGGCACTTTAGTTTTTGAGCTGCCGGACACCCGTAAG

AGTCTGGATCGCGAACGCGAATTCCCTGTGTGGAAGGAAGCAACCACCACTCTA
TCAGACCTAGCGCTTGCGCTTAAGGGACACACCTTCCTTCGTTGGTGGTGAGAT

TTTTGTGCATCAGATGCTAAAGCATATGATACAGAGGTACATAATGTTTGGGCC
AAAACACGTAGTCTACGATTTCGTATACTATGTCTCCATGTATTACAAACCCGG

ACACATGCCTGTGTACCCACAGACCCCAACCCACAAGAAGTAGTATTGGTAAAT
TGTGTACGGACACATGGGTGTCTGGGGTTGGGTGTTCTTCATCATAACCATTTA

GTGACAGAAAATTTTAACATGTGGAAAAATGACATGGTAGAACAGATGCATGAG
CACTGTCTTTTAAAATTGTACACCTTTTTACTGTACCATCTTGTCTACGTACTC

GATATAATCAGTTTATGGGATCAAAGCCTAAAGCCATGTGTAAAATTAACCCCA
CTATATTAGTCAAATACCCTAGTTTCGGATTTCGGTACACATTTTAATTGGGGT

CTCTGTGTTAGTTTAAAGTGCACTGATTTGAAGAATGATACTAATACCAATAGT
GAGACACAATCAAATTTCACGTGACTAAACTTCTTACTATGATTATGGTTATCA

AGTAGCGGGAGAATGATAATGGAGAAAGGAGAGATAAAAAACTGCTCTTTCAAT
TCATCGCCCTCTTACTATTACCTCTTTCCTCTCTATTTTTTGACGAGAAAGTTA

ATCAGCACAAGCATAAGAGGTAAGGTGCAGAAAGAATATGCATTTTTTTATAAA
TAGTCGTGTTCGTATTCTCCATTCCACGTCTTTCTTATACGTAAAAAAATATTT

CTTGATATAATACCAATAGATAATGATACTACCAGCTATACGTTGACAAGTTGT
GAACTATATTATGGTTATCTATTACTATGATGGTCGATATGCAACTGTTCAACA

AACACCTCAGTCATTACACAGGCCTGTCCAAAGGTATCCTTTGAGCCAATTCCC
TTGTGGAGTCAGTAATGTGTCCGGACAGGTTTCCATAGGAAACTCGGTTAAGGG

ATACATTATTGTGCCCCGGCTGGTTTTGCGATTCTAAAATGTAATAATAAGACG
TATGTAATAACACGGGGCCGACCAAAACGCTAAGATTTTACATTATTATTCTGC

TTCAATGGAACAGGACCATGTACAAATGTCAGCACAGTACAATGTACACATGGA
AAGTTACCTTGTCCTGGTACATGTTTACAGTCGTGTCATGTTACATGTGTACCT

ATTAGGCCAGTAGTATCAACTCAACTGCTGTTAAATGGCAGTCTAGCAGAAGAA
TAATCCGGTCATCATAGTTGAGTTGACGACAATTTACCGTCAGATCGTCTTCTT

GAGGTAGTAATTAGATCTGCCAATTTCACAGACAATGCTAAAACCATAATAGTA
CTCCATCATTAATCTAGACGGTTAAAGTGTCTGTTACGATTTTGGTATTATCAT

CAGCTGAACCAATCTGTAGAAATTAATTGTACAAGACCCAACAACAATACAAGA
GTCGACTTGGTTAGACATCTTTAATTAACATGTTCTGGGTTGTTGTTATGTTCT

AAAAGTATCCGTATCCAGAGAGGACCAGGGAGAGCATTTGTTACAATAGGAAAA
TTTTCATAGGCATAGGTCTCTCCTGGTCCCTCTCGTAAACAATGTTATCCTTTT
```

42 ATAGGAAATATGAGACAAGCACATTGTAACATTAGTAGAGCAAAATGGAATAAC
43 TATCCTTTATACTCTGTTCGTGTAACATTGTAATCATCTCGTTTTACCTTATTG

44 ACTTTAAAACAGATAGATAGCAAATTAAGAGAACAATTTGGAAATAATAAAACA
45 TGAAATTTTGTCTATCTATCGTTTAATTCTCTTGTTAAACCTTTATTATTTTGT

46 ATAATCTTTAAGCAGTCCTCAGGAGGGGACCCAGAAATTGTAACGCACAGTTTT
47 TATTAGAAATTCGTCAGGAGTCCTCCCCTGGGTCTTTAACATTGCGTGTCAAAA

48 AATTGTGGAGGGGAATTTTTCTACTGTAATTCAACACAACTGTTTAATAGTACT
49 TTAACACCTCCCCTTAAAAAGATGACATTAAGTTGTGTTGACAAATTATCATGA

50 TGGTTTAATAGTACTTGGAGTACTAAAGGGTCAAATAACACTGAAGGAAGTGAC
51 ACCAAATTATCATGAACCTCATGATTTCCCAGTTTATTGTGACTTCCTTCACTG

52 ACAATCACCCTCCCATGCAGAATAAAACAAATTATAAACATGTGGCAGGAAGTA
53 TGTTAGTGGGAGGGTACGTCTTATTTTGTTTAATATTTGTACACCGTCCTTCAT

54 GGAAAAGCAATGTATGCCCCTCCCATCAGTGGACAAATTAGATGTTCATCAAAT
55 CCTTTTCGTTACATACGGGGAGGGTAGTCACCTGTTTAATCTACAAGTAGTTTA

56 ATTACAGGGCTGCTATTAACAAGAGATGGTGGTAATAGCAACAATGAGTCCGAG
57 TAATGTCCCGACGATAATTGTTCTCTACCACCATTATCGTTGTTACTCAGGCTC

58 ATCTTCAGACCTGGAGGAGGAGATATGAGGGACAATTGGAGAAGTGAATTATAT
59 TAGAAGTCTGGACCTCCTCCTCTATACTCCCTGTTAACCTCTTCACTTAATATA

60 AAATATAAAGTAGTAAAAATTGAACCATTAGGAGTAGCACCCACCAAGGCAAAG
61 TTTATATTTCATCATTTTTAACTTGGTAATCCTCATCGTGGGTGGTTCCGTTTC

62 AGAAGAGTGGTGCAGAGAGAAAAAAGAGCAGTGGGAATAGGAGCTTTGTTCCTT
63 TCTTCTCACCACGTCTCTCTTTTTTCTCGTCACCCTTATCCTCGAAACAAGGAA

64 GGGTTCTTGGGAGCAGCAGGAAGCACTATGGGCGCAGCGTCAATGACGCTGACG
65 CCCAAGAACCCTCGTCGTCCTTCGTGATACCCGCGTCGCAGTTACTGCGACTGC

66 GTACAGGCCAGACAATTATTGTCTGGTATAGTGCAGCAGCAGAACAATTTGCTG
67 CATGTCCGGTCTGTTAATAACAGACCATATCACGTCGTCGTCTTGTTAAACGAC

68 AGGGCTATTGAGGCGCAACAGCATCTGTTGCAACTCACAGTCTGGGGCATCAAG
69 TCCCGATAACTCCGCGTTGTCGTAGACAACGTTGAGTGTCAGACCCCGTAGTTC

70 CAGCTCCAGGCAAGAATCCTGGCTGTGGAAAGATACCTAAAGGATCAACAGCTC
71 GTCGAGGTCCGTTCTTAGGACCGACACCTTTCTATGGATTTCCTAGTTGTCGAG

72 CTGGGGATTTGGGGTTGCTCTGGAAAACTCATTTGCACCACTGCTGTGCCTTGG
73 GACCCCTAAACCCCAACGAGACCTTTTGAGTAAACGTGGTGACGACACGGAACC

74 AATGCTAGTTGGAGTAATAAATCTCTGGAACAGATTTGGAATAACATGACCTGG
75 TTACGATCAACCTCATTATTTAGAGACCTTGTCTAAACCTTATTGTACTGGACC

76 ATGGAGTGGGACAGAGAAATTAACAATTACACAAGCTTCCCGGGAGCTCGAATT
77 TACCTCACCCTGTCTCTTTAATTGTTAATGTGTTCGAAGGGCCCTCGAGCTTAA

78 CTTGAAGACGAAAGGGCCTCG
79 GAACTTCTGCTTTCCCGGAGC.

5. The DNA transfer vector of any preceding claim transferred to and replicated in a eukaryotic or prokaryotic host.

6. A host transformed by the transfer vector of any of claims 1 to 4.

7. HTLV-III envelope protein fragment denoted R10 having the following amino-acid sequence, or mutants thereof:

MetLeuArg
ProValGluThrProThrArgGluIleLysLysLeuAspGlyLeuTrpAlaPhe
SerLeuAspArgGluAsnCysGlyIleAspGlnPheProValTrpLysGluAla
ThrThrThrLeuPheCysAlaSerAspAlaLysAlaTyrAspThrGluValHis
AsnValTrpAlaThrHisAlaCysValProThrAspProAsnProGlnGluVal
ValLeuValAsnValThrGluAsnPheAsnMetTrpLysAsnAspMetValGlu

GlnMetHisGluAspIleIleSerLeuTrpAspGlnSerLeuLysProCysVal
LysLeuThrProLeuCysValSerLeuLysCysThrAspLeuLysAsnAspThr
AsnThrAsnSerSerSerGlyArgMetIleMetGluLysGlyGluIleLysAsn
CysSerPheAsnIleSerThrSerIleArgGlyLysValGlnLysGluTyrAla
PhePheTyrLysLeuAspIleIleProIleAspAsnAspThrThrSerTyrThr
LeuThrSerCysAsnThrSerValIleThrGlnAlaCysProLysValSerPhe
GluProIleProIleHisTyrCysAlaProAlaGlyPheAlaIleLeuLysCys
AsnAsnLysThrPheAsnGlyThrGlyProCysThrAsnValSerThrValGln
CysThrHisGlyIleArgProValValSerThrGlnLeuLeuLeuAsnGlySer
LeuAlaGluGluGluValValIleArgSerAlaAsnPheThrAspAsnAlaLys
ThrIleIleValGlnLeuAsnGlnSerValGluIleAsnCysThrArgProAsn
AsnAsnThrArgLysSerIleArgIleGlnArgGlyProGlyArgAlaPheVal
ThrIleGlyLysIleGlyAsnMetArgGlnAlaHisCysAsnIleSerArgAla
LysTrpAsnAsnThrLeuLysGlnIleAspSerLysLeuArgGluGlnPheGly
AsnAsnLysThrIleIlePheLysGlnSerSerGlyGlyAspProGluIleVal
ThrHisSerPheAsnCysGlyGlyGluPhePheTyrCysAsnSerThrGlnLeu
PheAsnSerThrTrpPheAsnSerThrTrpSerThrLysGlySerAsnAsnThr
GluGlySerAspThrIleThrLeuProCysArgIleLysGlnIleIleAsnMet
TrpGlnGluValGlyLysAlaMetTyrAlaProProIleSerGlyGlnIleArg
CysSerSerAsnIleThrGlyLeuLeuLeuThrArgAspGlyGlyAsnSerAsn
AsnGluSerGluIleHisArgSerValMetLeuTyrThrThrProAsnThrTrp
ValAspAspIleThrValValThrHisValAlaGlnAspCysAsnHisAlaSer
ValAspTrpGlnValValAlaAsnGlyAspValSerValGluLeuArgAspAla
AspGlnGlnValValAlaThrGlyGlnGlyThrSerGlyThrLeuGlnValVal
AsnProHisLeuTrpGlnProGlyGluGlyTyrLeuTyrGluLeuCysValThr
AlaLysSerGlnThrGluCysAspIleTyrProLeuArgValGlyIleArgSer

ValAlaValLysGlyGluGlnPheLeuIleAsnHisLysProPheTyrPheThr
GlyPheGlyArgHisGluAspAlaAspLeuArgGlyLysGlyPheAspAsnVal
LeuMetValHisAspHisAlaLeuMetAspTrpIleGlyAlaAsnSerTyrArg
ThrSerHisTyrProTyrAlaGluGluMetLeuAspTrpAlaAspGluHisGly
IleValValIleAspGluThrAlaAlaValGlyPheAsnLeuSerLeuGlyIle
GlyPheGluAlaGlyAsnLysProLysGluLeuTyrSerGluGluAlaValAsn
GlyGluThrGlnGlnAlaHisLeuGlnAlaIleLysGluLeuIleAlaArgAsp
LysAsnHisProSerValValMetTrpSerIleAlaAsnGluProAspThrArg
ProGlnGlyAlaArgGluTyrPheAlaProLeuAlaGluAlaThrArgLysLeu
AspProThrArgProIleThrCysValAsnValMetPheCysAspAlaHisThr
AspThrIleSerAspLeuPheAspValLeuCysLeuAsnArgTyrTyrGlyTrp
TyrValGlnSerGlyAspLeuGluThrAlaGluLysValLeuGluLysGluLeu
LeuAlaTrpGlnGluLysLeuHisGlnProIleIleIleThrGluTyrGlyVal
AspThrLeuAlaGlyLeuHisSerMetTyrThrAspMetTrpSerGluGluTyr
GlnCysAlaTrpLeuAspMetTyrHisArgValPheAspArgValSerAlaVal
ValGlyGluGlnValTrpAsnPheAlaAspPheAlaThrSerGlnGlyIleLeu
ArgValGlyGlyAsnLysLysGlyIlePheThrArgAspArgLysProLysSer
AlaAlaPheLeuLeuGlnLysArgTrpThrGlyMetAsnPheGlyGluLysPro
GlnGlnGlyGlyLysGln.

8. HTLV-III envelope protein fragment denoted PB1 having the following amino-acid sequence, or mutants thereof:

MetLeuArg
ProValGluThrProThrArgGluIleLysLysLeuAspGlyLeuTrpAlaPhe
SerLeuAspArgGluArgValAlaAspLeuAsnGlnSerValGluIleAsnCys
ThrArgProAsnAsnAsnThrArgLysSerIleArgIleGlnArgGlyProGly

ArgAlaPheValThrIleGlyLysIleGlyAsnMetArgGlnAlaHisCysAsn
IleSerArgAlaLysTrpAsnAsnThrLeuLysGlnIleAspSerLysLeuArg
GluGlnPheGlyAsnAsnLysThrIleIlePheLysGlnSerSerGlyGlyAsp
ProGluIleValThrHisSerPheAsnCysGlyGlyGluPhePheTyrCysAsn
SerThrGlnLeuPheAsnSerThrTrpPheAsnSerThrTrpSerThrLysGly
SerAsnAsnThrGluGlySerAspThrIleThrLeuProCysArgIleLysGln
IleIleAsnMetTrpGlnGluValGlyLysAlaMetTyrAlaProProIleSer
GlyGlnIleArgCysSerSerAsnIleThrGlyLeuLeuLeuThrArgAspGly
GlyAsnSerAsnAsnGluSerGluIleArgArgGlnAlaSerArgGluLeuGlu
PheLeuLysThrLysGlyProArgAspThrProIlePheIleGly.

9. HTLV-III envelope protein fragment denoted 590 having the following amino-acid sequence, or mutants thereof:

MetLeuArgProValGluThr
ProThrArgGluIleLysLysLeuAspGlyLeuTrpAlaPheSerLeuAspArg
GluArgValAlaAspLeuAsnGlnSerValGluIleAsnCysThrArgProAsn
AsnAsnThrArgLysSerIleArgIleGlnArgGlyProGlyArgAlaPheVal
ThrIleGlyLysIleGlyAsnMetArgGlnAlaHisCysAsnIleSerArgAla
LysTrpAsnAsnThrLeuLysGlnIleAspSerLysLeuArgGluGlnPheGly
AsnAsnLysThrIleIlePheLysGlnSerSerGlyGlyAspProGluIleVal
ThrHisSerPheAsnCysGlyGlyGluPhePheTyrCysAsnSerThrGlnLeu
PheAsnSerThrTrpPheAsnSerThrTrpSerThrLysGlySerAsnAsnThr
GluGlySerAspThrIleThrLeuProCysArgIleLysGlnIleIleAsnMet
TrpGlnGluValGlyLysAlaMetTyrAlaProProIleSerGlyGlnIleArg
CysSerSerAsnIleThrGlyLeuLeuLeuThrArgAspGlyGlyAsnSerAsn
AsnGluSerGluIlePheArgProGlyGlyGlyAspMetArgAspAsnTrpArg
SerGluLeuTyrLysTyrLysValValLysIleGluProLeuGlyValAlaPro

ThrLysAlaLysArgArgValValGlnArgGluLysArgAlaValGlyIleGly
AlaLeuPheLeuGlyPheLeuGlyAlaAlaGlySerThrMetGlyAlaAlaSer
MetThrLeuThrValGlnAlaArgGlnLeuLeuSerGlyIleValGlnGlnGln
AsnAsnLeuLeuArgAlaIleGluAlaGlnGlnHisLeuLeuGlnLeuThrVal
TrpGlyIleLysGlnLeuGlnAlaArgIleLeuAlaValGluArgTyrLeuLys
AspGlnGlnLeuLeuGlyIleTrpGlyCysSerGlyLysLeuIleCysThrThr
AlaValProTrpAsnAlaSerTrpSerAsnLysSerLeuGluGlnIleTrpAsn
AsnMetThrTrpMetGluTrpAspArgGluIleAsnAsnTyrThrSerPhePro
IleHisArgSerValMetLeuTyrThrThrProAsnThrTrpValAspAspIle
ThrValValThrHisValAlaGlnAspCysAsnHisAlaSerValAspTrpGln
ValValAlaAsnGlyAspValSerValGluLeuArgAspAlaAspGlnGlnVal
ValAlaThrGlyGlnGlyThrSerGlyThrLeuGlnValValAsnProHisLeu
TrpGlnProGlyGluGlyTyrLeuTyrGluLeuCysValThrAlaLysSerGln
ThrGluCysAspIleTyrProLeuArgValGlyIleArgSerValAlaValLys
GlyGluGlnPheLeuIleAsnHisLysProPheTyrPheThrGlyPheGlyArg
HisGluAspAlaAspLeuArgGlyLysGlyPheAspAsnValLeuMetValHis
AspHisAlaLeuMetAspTrpIleGlyAlaAsnSerTyrArgThrSerHisTyr
ProTyrAlaGluGluMetLeuAspTrpAlaAspGluHisGlyIleValValIle
AspGluThrAlaAlaValGlyPheAsnLeuSerLeuGlyIleGlyPheGluAla
GlyAsnLysProLysGluLeuTyrSerGluGluAlaValAsnGlyGluThrGln
GlnAlaHisLeuGlnAlaIleLysGluLeuIleAlaArgAspLysAsnHisPro
SerValValMetTrpSerIleAlaAsnGluProAspThrArgProGlnGlyAla
ArgGluTyrPheAlaProLeuAlaGluAlaThrArgLysLeuAspProThrArg
ProIleThrCysValAsnValMetPheCysAspAlaHisThrAspThrIleSer
AspLeuPheAspValLeuCysLeuAsnArgTyrTyrGlyTrpTyrValGlnSer
GlyAspLeuGluThrAlaGluLysValLeuGluLysGluLeuLeuAlaTrpGln
GluLysLeuHisGlnProIleIleIleThrGluTyrGlyValAspThrLeuAla

GlyLeuHisSerMetTyrThrAspMetTrpSerGluGluTyrGlnCysAlaTrp
LeuAspMetTyrHisArgValPheAspArgValSerAlaValValGlyGluGln
ValTrpAsnPheAlaAspPheAlaThrSerGlnGlyIleLeuArgValGlyGly
AsnLysLysGlyIlePheThrArgAspArgLysProLysSerAlaAlaPheLeu
LeuGlnLysArgTrpThrGlyMetAsnPheGlyGluLysProGlnGlnGlyGly
LysGln.

10. HTLV-III envelope protein fragment denoted KH1 having the following amino-acid sequence, or mutants thereof:

MetLeuArg
ProValGluThrProThrArgGluIleLysLysLeuAspGlyLeuTrpAlaPhe
SerLeuAspArgGluArgGluPheProValTrpLysGluAlaThrThrThrLeu
PheCysAlaSerAspAlaLysAlaTyrAspThrGluValHisAsnValTrpAla
ThrHisAlaCysValProThrAspProAsnProGlnGluValValLeuValAsn
ValThrGluAsnPheAsnMetTrpLysAsnAspMetValGluGlnMetHisGlu
AspIleIleSerLeuTrpAspGlnSerLeuLysProCysValLysLeuThrPro
LeuCysValSerLeuLysCysThrAspLeuLysAsnAspThrAsnThrAsnSer
SerSerGlyArgMetIleMetGluLysGlyGluIleLysAsnCysSerPheAsn
IleSerThrSerIleArgGlyLysValGlnLysGluTyrAlaPhePheTyrLys
LeuAspIleIleProIleAspAsnAspThrThrSerTyrThrLeuThrSerCys
AsnThrSerValIleThrGlnAlaCysProLysValSerPheGluProIlePro
IleHisTyrCysAlaProAlaGlyPheAlaIleLeuLysCysAsnAsnLysThr
PheAsnGlyThrGlyProCysThrAsnValSerThrValGlnCysThrHisGly
IleArgProValValSerThrGlnLeuLeuLeuAsnGlySerLeuAlaGluGlu
GluValValIleArgSerAlaAsnPheThrAspAsnAlaLysThrIleIleVal
GlnLeuAsnGlnSerValGluIleAsnCysThrArgProAsnAsnAsnThrArg
LysSerIleArgIleGlnArgGlyProGlyArgAlaPheValThrIleGlyLys

IleGlyAsnMetArgGlnAlaHisCysAsnIleSerArgAlaLysTrpAsnAsn ThrLeuLysGlnIleAspSerLysLeuArgGluGlnPheGlyAsnAsnLysThr IleIlePheLysGlnSerSerGlyGlyAspProGluIleValThrHisSerPhe AsnCysGlyGlyGluPhePheTyrCysAsnSerThrGlnLeuPheAsnSerThr TrpPheAsnSerThrTrpSerThrLysGlySerAsnAsnThrGluGlySerAsp ThrIleThrLeuProCysArgIleLysGlnIleIleAsnMetTrpGlnGluVal GlyLysAlaMetTyrAlaProProIleSerGlyGlnIleArgCysSerSerAsn IleThrGlyLeuLeuLeuThrArgAspGlyGlyAsnSerAsnAsnGluSerGlu IlePheArgProGlyGlyGlyAspMetArgAspAsnTrpArgSerGluLeuTyr LysTyrLysValValLysIleGluProLeuGlyValAlaProThrLysAlaLys ArgArgValValGlnArgGluLysArgAlaValGlyIleGlyAlaLeuPheLeu GlyPheLeuGlyAlaAlaGlySerThrMetGlyAlaAlaSerMetThrLeuThr ValGlnAlaArgGlnLeuLeuSerGlyIleValGlnGlnGlnAsnAsnLeuLeu ArgAlaIleGluAlaGlnGlnHisLeuLeuGlnLeuThrValTrpGlyIleLys GlnLeuGlnAlaArgIleLeuAlaValGluArgTyrLeuLysAspGlnGlnLeu LeuGlyIleTrpGlyCysSerGlyLysLeuIleCysThrThrAlaValProTrp AsnAlaSerTrpSerAsnLysSerLeuGluGlnIleTrpAsnAsnMetThrTrp MetGluTrpAspArgGluIleAsnAsnTyrThrSerPheProGlyAlaArgIle LeuGluAspGluArgAlaSer.

11. A plasmid selected from the following: plasmid pREV1, plasmid pREV1TT, plasmid pREV1TT/ch1, plasmid pREV2.2, plasmid pR10, plasmid pPB1, plasmid p590, and plasmid pKH1, and preferably any of the last five of these eight plasmids.

12. DNA having the nucleotide sequence defined in any of claims 1 to 4, or an equivalent nucleotide sequence containing bases whose translated region codes for HTLV-III envelope protein fragment denoted R10, PB1, 590 or KH1.

13. An immunochemical assay for detecting or quantifying antibody against HTLV-III in a fluid, which comprises employing an HTLV-III protein selected from R10, PB1, 590 and KH1.

14. An immunoadsorbent suitable for use in a solid phase immunochemical assay for antibody against HTLV-III, which comprises a solid phase to which is affixed an HTLV-III protein selected from R10, PB1, 590 and KH1.

15. An immunoadsorbent according to claim 14, wherein the solid phase is a glass or plastic bead, a well of a microtiter plate or a test tube.

16. An immunoadsorbent according to claim 14 or claim 15, which additionally comprises a post-coat of animal protein.

17. A kit suitable for use in detecting antibody against HTLV-III in a biological fluid, which comprises:

(a) an immunoadsorbent according to any of claims 14 to 16;

(b) labelled HTLV-III antibody; and

(c) means for detecting the label associated with the immunoadsorbent.

18. A kit according to claim 17, wherein the anti-HTLV-III antibody is labelled with anti-(human IgG) antibody.

19. A method of detecting antibody against HTLV-III in a biological fluid, which comprises the steps of:

(a) incubating an immunoadsorbent according to any of claims 14 to 16 with a sample of the biological fluid, under conditions which allow anti-HTLV-III antibody in the sample to bind to the immunoadsorbent;

(b) separating the immunoadsorbent from the sample; and

(c) determining if antibody has bound to the immunoadsorbent as an indication of anti-HTLV-III in the sample.

20. A method according to claim 19, wherein step (c) comprises incubating the immunoadsorbent with (i) a labelled antibody against antigen of the species from which the biological fluid is derived, (ii) labelled HTLV-III protein selected from R10, PB1, 590 and KH1, or (iii) labelled protein A; separating the immunoadsorbent from the labelled antibody, HTLV-III protein or protein A; and detecting the label associated with the immunoadsorbent.

21. A method of detecting antibody against HTLV-III in a human serum or plasma sample, which comprises the steps of:

(a) incubating a bead of an immunoadsorbent according to any of claims 14 to 16 with the serum or plasma sample under conditions which allow anti-HTLV-III antibody in the sample to bind to the immunoadsorbent;

(b) separating the immunoadsorbent and the sample;

(c) incubating the immunoadsorbent with a labelled anti-(human IgG) antibody under conditions which allow the anti-(human IgG) antibody to bind human anti-HTLV-III antibody bound to the immunoadsorbent;

(d) separating the immunoadsorbent from the unbound anti-(human IgG) antibody; and

(e) evaluating the label associated with the immunoadsorbent as an indication of the presence of antibody against HTLV-III in the sample.

22. A method according to claim 21, wherein the anti-(human IgG) antibody is an animal antibody and the serum or plasma sample is diluted with normal serum of an animal of the same species.

23. A method according to claim 22, wherein the anti-(human IgG) antibody is a goat antibody and the serum or plasma sample is diluted with normal goat serum.

24. A method according to any of claims 21 to 23, wherein the anti-(human IgG) antibody is labelled with a radioisotope, an enzyme or a fluorescent compound.

25. A vaccine composition which comprises an HTLV-III protein having the antigenic properties of R10, PB1, 590 or KH1, in a pharmacologically-acceptable vehicle.

26. A recombinant HTLV-III envelope protein fragment selected from R10, PB1, 590 and KH1, for therapeutic use.

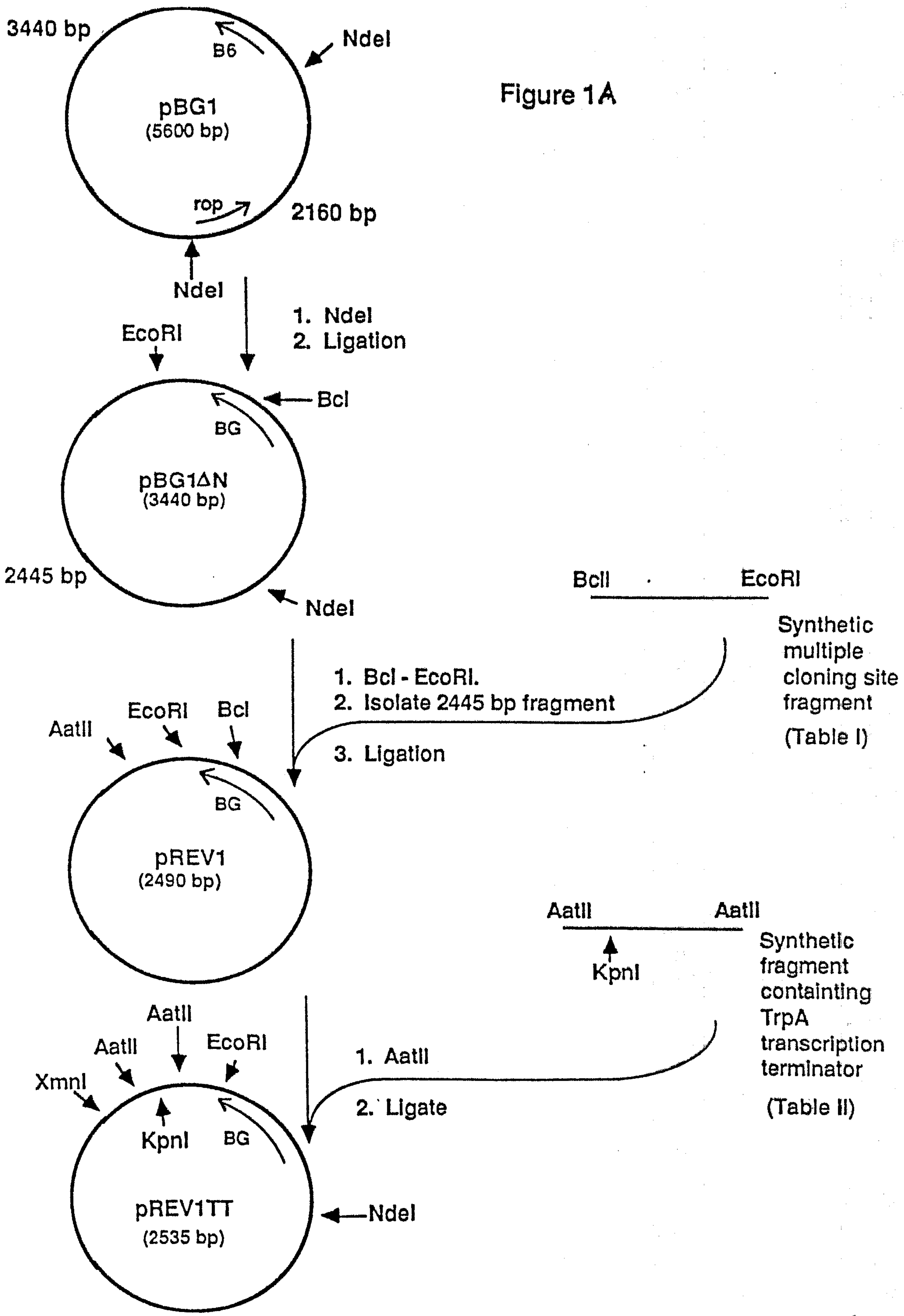
3440 bp
B6
NdeI
pBG1
(5600 bp)
rop
2160 bp
NdeI
1. NdeI
2. Ligation
EcoRI
BcI
BG
pBG1ΔN
(3440 bp)
2445 bp
NdeI
BclI
EcoRI
Synthetic
multiple
cloning site
fragment
(Table I)
1. Bcl - EcoRI.
2. Isolate 2445 bp fragment
3. Ligation
AatII
EcoRI
Bcl
BG
pREV1
(2490 bp)
AatII
AatII
KpnI
Synthetic
fragment
containting
TrpA
transcription
terminator
(Table II)
AatII
AatII
EcoRI
XmnI
1. AatII
2. Ligate
KpnI
BG
pREV1TT
(2535 bp)
NdeI

Figure 1A

Figure 1B

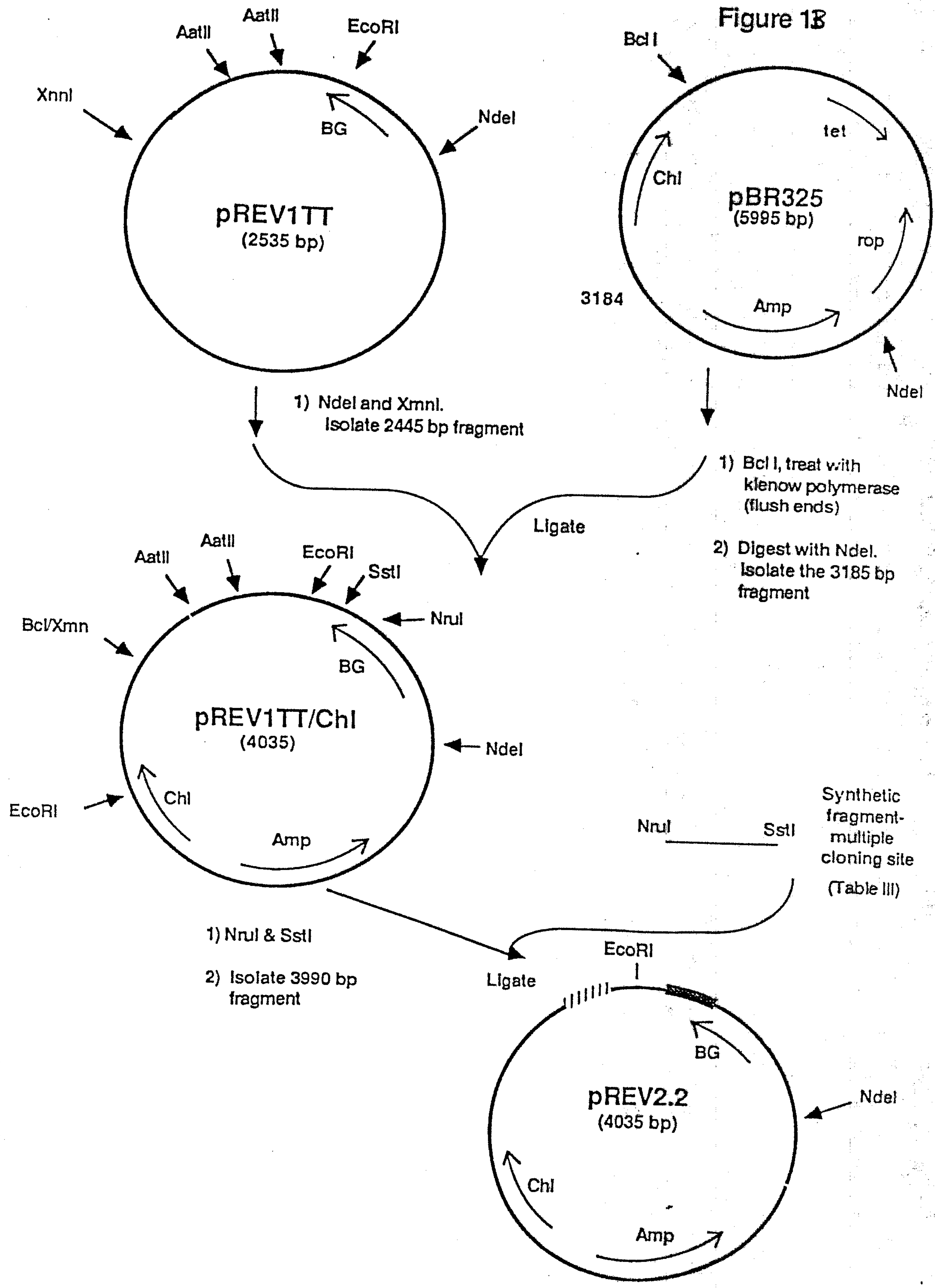
AatII
AatII
EcoRI
XnnI
NdeI
BG
pREV1TT
(2535 bp)
BcI I
Chl
tet
pBR325
(5995 bp)
rop
3184
Amp
NdeI
1) NdeI and XmnI.
Isolate 2445 bp fragment
1) BcI I, treat with klenow polymerase (flush ends)
2) Digest with NdeI.
Isolate the 3185 bp fragment
Ligate
AatII
AatII
EcoRI
SstI
NruI
Bcl/Xmn
BG
pREV1TT/Chl
(4035)
NdeI
EcoRI
Chl
Amp
NruI
SstI
Synthetic fragment-multiple cloning site
(Table III)
1) NruI & SstI
2) Isolate 3990 bp fragment
Ligate
EcoRI
BG
pREV2.2
(4035 bp)
NdeI
Chl
Amp

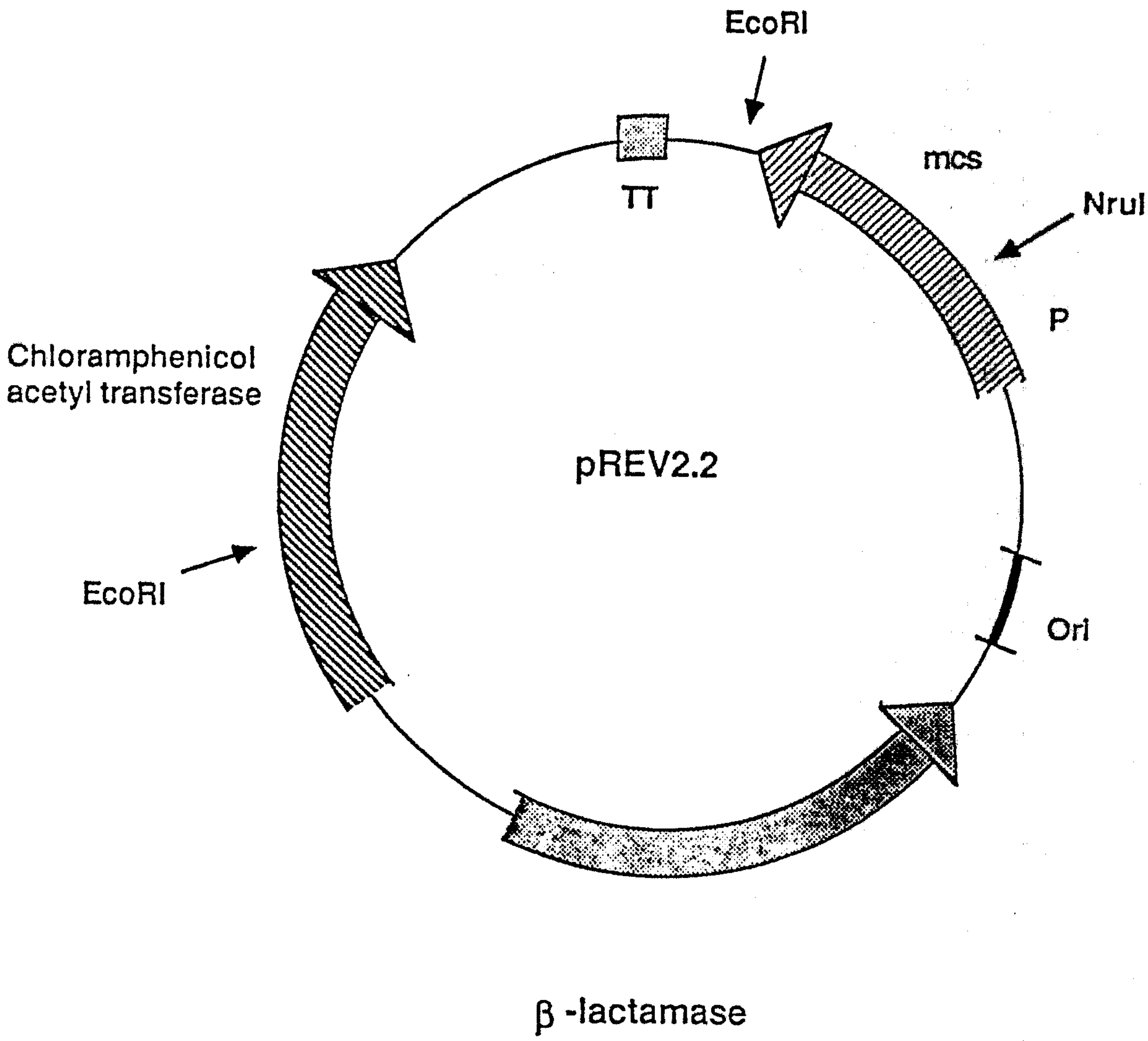

EcoRI
TT
mcs
NruI
P
Chloramphenicol acetyl transferase
pREV2.2
EcoRI
Ori
β -lactamase


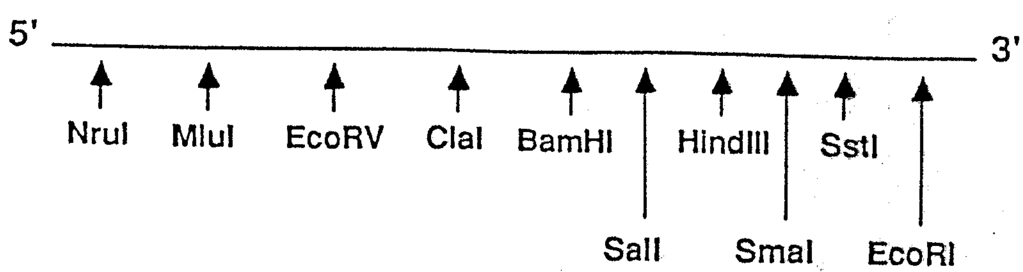

5'
3'
NruI
MluI
EcoRV
ClaI
BamHI
HindIII
SstI
SalI
SmaI
EcoRI


Figure 2

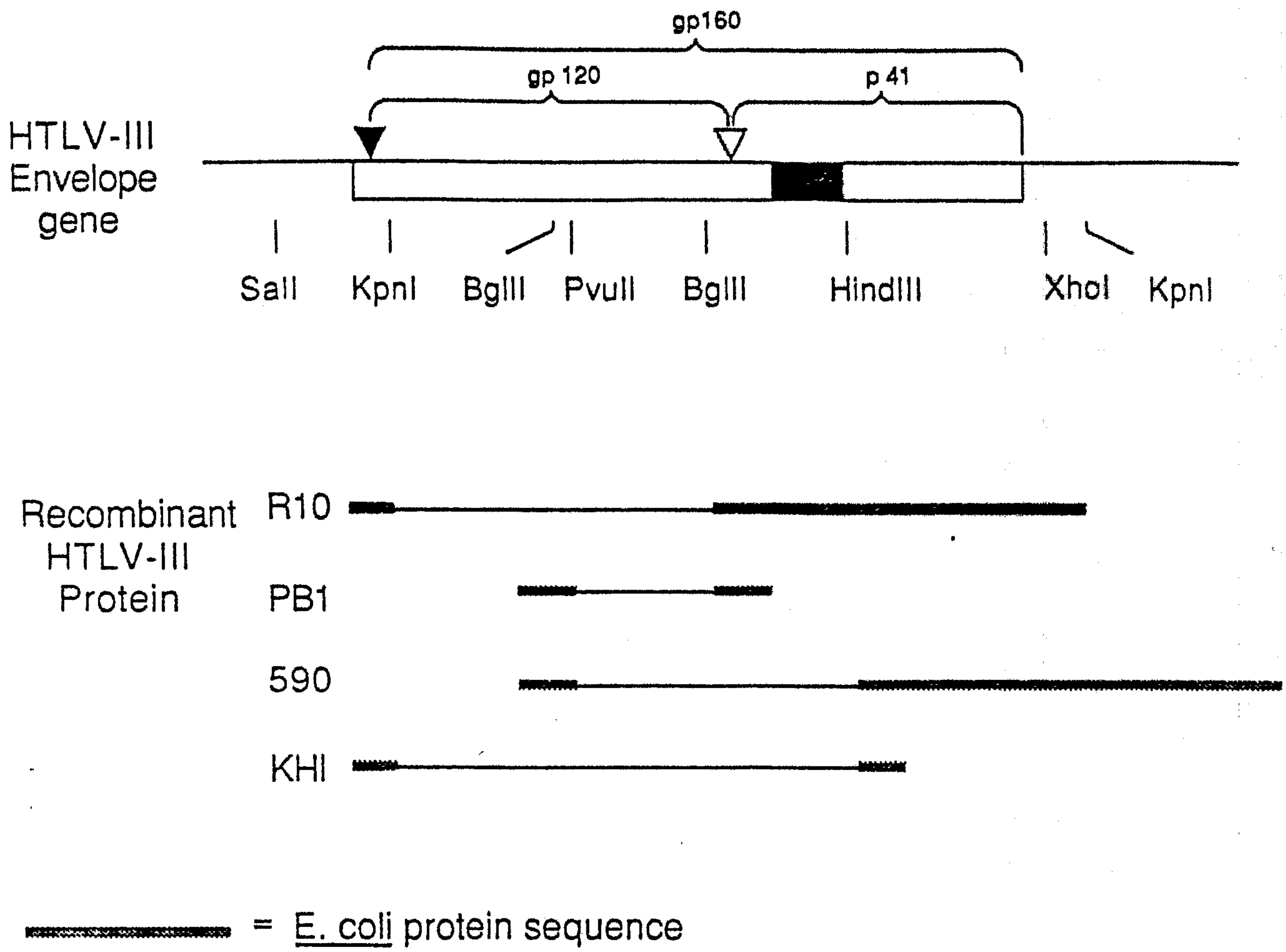
gp160
gp 120
p 41
HTLV-III Envelope gene
SalI
KpnI
BglII
PvuII
BglII
HindIII
XhoI
KpnI
Recombinant HTLV-III Protein
R10
PB1
590
KHI
= E. coli protein sequence

Figure 3

European Patent Office

Application number:

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:

NRRL B-18091

NRRL B-18093

NRRL B-18092

NRRL B-18094

NRRL B-18095